# EUROPEAN PATENT SPECIFICATION

(11) **EP 2 991 979 B1**
(45) Date of publication and mention of the grant of the patent: **07.06.2017**
(21) Application number: 15715244.8
(22) Date of filing: 08.04.2015
(51) Int. Cl.: C07D 405/04, A61K 31/505

(54) **CO-CRYSTALS OF LAPATINIB MONOACID SALTS**
CO-KRISTALLE VON LAPATINIB-MONOSÄURESALZEN
COCRISTAUX DE SELS MONOACIDES DE LAPATINIB

(30) Priority: 24.04.2014 EP 14165887
(43) Date of publication of application: 09.03.2016
(73) Proprietor: F.I.S.- Fabbrica Italiana Sintetici S.p.A., 36075 Alte di Montecchio Maggiore (VI) (IT)
(72) Inventor: TESSON, Nicolas, 08028 Barcelona (ES); SEGADE RODRÍGUEZ, Antonio, 08017 Barcelona (ES)
(74) Representative: Leganza, Alessandro
(86) International application number: PCT/EP2015/057630
(87) International publication number: WO 2015/162007

(56) References cited:
- WO-A2-2010/081443
- WO-A2-2014/016848
- US-A1- 2013 102 781
- US-B2- 7 927 613
- ROCHE S ET AL: "Development of a high-performance liquid chromatographic-mass spectrometric method for the determination of cellular levels of the tyrosine kinase inhibitors lapatinib and dasatinib", JOURNAL OF CHROMATOGRAPHY B: BIOMEDICAL SCIENCES & APPLICATIONS, ELSEVIER, AMSTERDAM, NL, vol. 877, no. 31, 1 December 2009 (2009-12-01), pages 3982-3990, XP026756479, ISSN: 1570-0232, DOI: 10.1016/J.JCHROMB.2009.10.008 [retrieved on 2009-10-12]
- "Pharmaceutical Salts", , 1 May 1958 (1958-05-01), pages 334-345, XP055136472, Retrieved from the Internet: URL:http://phoenix.tuwien.ac.at/pdf/pharma ceutical_salts/Pharmaceutical_salts.pdf [retrieved on 2014-08-26]
- XU LIU ET AL: "Improving the Chemical Stability of Amorphous Solid Dispersion with Cocrystal Technique by Hot Melt Extrusion", PHARMACEUTICAL RESEARCH, KLUWER ACADEMIC PUBLISHERS-PLENUM PUBLISHERS, NL, vol. 29, no. 3, 19 October 2011 (2011-10-19), pages 806-817, XP035016527, ISSN: 1573-904X, DOI: 10.1007/S11095-011-0605-4

## Description

### Technical Field

Object of the present invention are co-crystals, processes for the preparation thereof and uses of the active pharmaceutical ingredient named Lapatinib.

### Background Art

Lapatinib is an active pharmaceutical ingredient used for the treatment of breast cancer and other solid tumors. It is used for the treatment of patients with advanced or metastatic breast cancer whose tumors overexpress HER2 and is currently on the market under the name Tykerb. According to the manufacturer's instructions, the commercial product Tykerb contains Lapatinib as the ditosylate monohydrate salt of formula (Ibis):

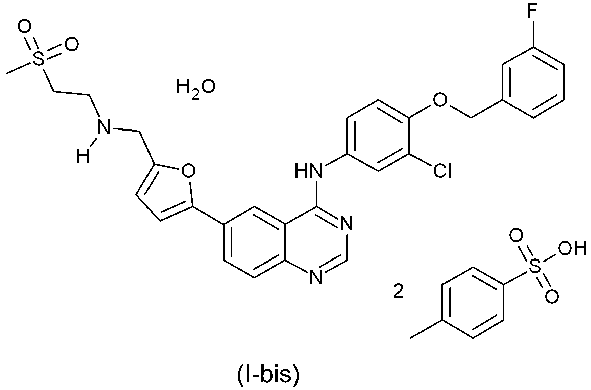

having the chemical name N-{3-chloro-4-[(3-fluorobenzyl)oxy]phenyl}-6-[5-({[2-(methylsulphonyl)ethyl]amino}methyl)furan-2-yl]quinazolin-4-amine bis(4-methylbenzenesulphonate) monohydrate, CAS RN 388082-78-8 and melting point 250-256°C.

The crystalline form of Lapatinib ditosylate monohydrate which, according to the statements of the author, appears to be that of the product currently on the market, is disclosed by Varlashkin P. on Power Diffr., Vol.24, No.3, September 2009, 250-253.

This substance can be prepared according to the teachings of the prior art such as, for example, those contained in EP1047694B1 or US7,157,466. In particular, in said last reference, examples 10 and 11 show the preparation of the ditosylate monohydrate salt starting from the anhydrous ditosylate salt.

Alternatively, Lapatinib and its ditosylate anhydrous and monohydrate form can be prepared according to the very efficient process described in the application EP13172771.1 and, moreover, the impurity orto-Lapatinib should be considered according to the teachings of EP2489661B.

In literature many solid forms of Lapatinib are disclosed, most of them are related to crystalline forms of Lapatinib ditosylate anhydrous (e.g. on IPCOM000199806 and IPCOM000204533) and few others are related to crystalline forms of Lapatinib base and Lapatinib ditosylate monohydrate. Moreover, many others solid forms of Lapatinib are disclosed being salts of Lapatinib (mainly disclosed on WO2010027848 and WO2008154469) and, furthermore, also are disclosed many solvates of Lapatinib ditosylate.

The application WO2010027848 has disclosed Lapatinib monotosylate, its crystalline form M1 and method for the preparation thereof.

Example 3 of WO2010/081443 discloses a co-crystal of Lapatinib ditosylate with guanidine hydrochloride and its preparation.

Lapatinib ditosylate monohydrate is a non-hygroscopic yellow crystalline solid. The choice of this salt has been justified based on crystalline properties and good bioavailability. It was convincingly demonstrated that the particle size did not significantly impact dissolution. It is practically insoluble in aqueous media. (Tyverb : EPAR - Public assessment report).

Ratain and Cohen showed that the bioavailability of the new anti-cancer agent, Lapatinib, increased 325% when co-administered with a high fat meal when compared with fasting conditions. Lapatinib is a Class II drug (low solubility - good permeability) (Custodio J.M. at al., Adv Drug Deliv Rev., 2008 March 17; 60(6), 717-733 and M.J. Ratain, E.E. Cohen, The value meal: how to save $1,700 per month or more on lapatinib, J. Clin. Oncol. 25 (2007) 3397-3398).

Thus, a big drawback of Lapatinib and the known solid forms is the relatively low bioavailability, due to the very low solubility in water. D2 discloses pharmaceutical co-crystal compositions. Lapatinb is specifically disclosed for instance in column 306. Caproic, caprylic and adipic acids are disclosed in columns 51-53 as co-crystal formers. D3 discloses in [0057]-[0060] co-crystal of lapatinib and anti-oxidative acids as co-formers.

### Summary of invention

The problem addressed by the present invention regards the provision of an improved solid form of Lapatinib, in particular, a solid form suitable for immediate release oral pharmaceutical forms, which allows at least partially overcoming the aforementioned drawbacks with reference to the prior art, especially those related to the low bioavailability of Lapatinib.

This problem is resolved through a solid form of Lapatinib as outlined in the attached claims, whose definitions are an integral part of the present description.

Further characteristics and advantages of the solid forms according to the invention will be apparent from the description - indicated hereinafter - of preferred embodiments, provided by means of non-limiting example.

### Brief description of drawings

Figure 1 shows the Differential Scanning Calorimetry (DSC) curve of the co-crystal Lapatinib monotosylate : caproic acid (1:2) of formula (Form I);
Figure 2 shows the X-Ray Powder Diffraction (XPRD) diffratogram of the co-crystal Lapatinib monotosylate : caproic acid (1:2) of formula (Form I);
Figure 3 shows the Differential Scanning Calorimetry (DSC) curve of the co-crystal Lapatinib monotosylate : caprylic acid (1:2) of formula (Form II);
Figure 4 shows the X-Ray Powder Diffraction (XPRD) diffratogram of the co-crystal Lapatinib monotosylate : caprylic acid (1:2) of formula (Form II);
Figure 7 shows Plasma Lapatinib concentration obtained after the intravenous administration of Lapatinib ditosylate monohydrate in DMSO:PEG:WFI (10:40:50) at 10 mg/kg to male rats (Group 1) semilogarithmic plot.
Figure 8 shows the individual and mean plasma Lapatinib concentrations obtained after its oral administration as a ditosylate monohydrate (comparative reference form) or as three different solid co-crystal formulations in hydroxypropylmethylcellulose : Tween 80 (0.5%:0.1%) in WFI at 30 mg/kg to male rats represented in a linear (top) and in a semi-logarithmic (bottom) plot.
Figure 9 shows the Pharmacokinetic model after Oral administration.
Figure 10 show the scatter plot of the observed data with the model prediction after IV administration.
Figure 11 show the scatter plot of the observed data with the model prediction after oral administration by treatment group.

### Description of embodiments

The present invention concerns co-crystal of Lapatinib monoacid salt selected in the group consisting of:
(a) Lapatinib monotosylate : caproic acid (1:2) of formula (Form I): 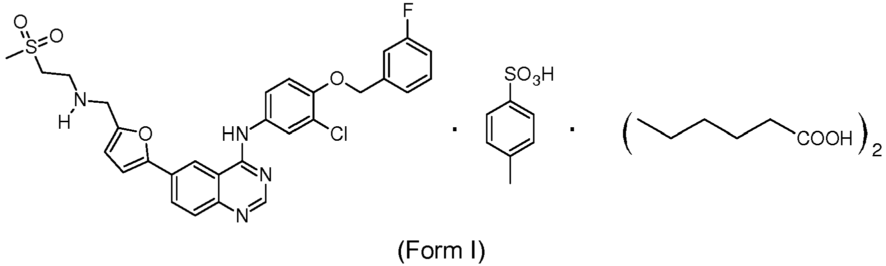
(b) Lapatinib monotosylate : caprylic acid (1:2) of formula (Form II): 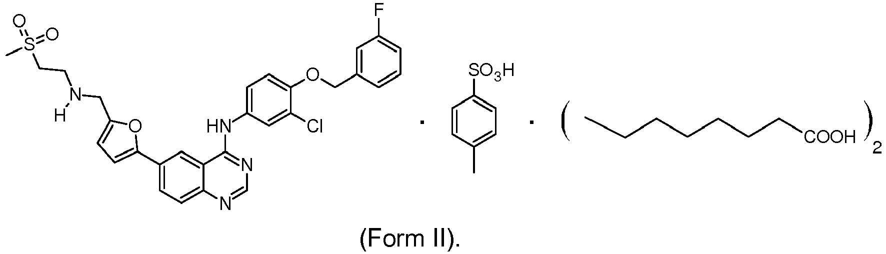 An other co-crystal of Lapatinib monoacid salt, which is not part of the present invention, is Lapatinib monohydrochloride : adipic acid (1:1) of formula (Form III):

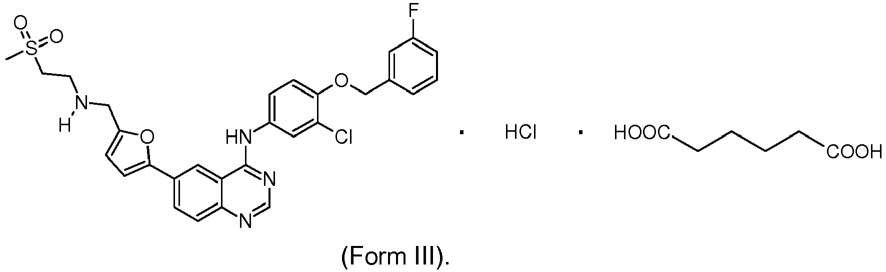

It has been indeed surprisingly found that is possible to prepare and isolate stable co-crystals of Lapatinib, being in form of Lapatinib monoacid salt.

The large amount of publications directed to solid forms of Lapatinib testifies that a huge effort in terms of R&D has been carried out by many companies to discover new solid forms of Lapatinib, nevertheless, co-crystals of Lapatinib as monoacid salts, including co-crystals of Lapatinib monotosylate or of Lapatinib monohydrochloride appear not to be known up to now.

A co-crystal can be described as a crystalline structure formed by two different or more molecular entities where, in contrast to salts, the intermolecular interactions are not ionic, but weak forces like hydrogen bonding and π-π stacking.

In terms of general knowledge, the formation of co-crystals presents a highly unexpected and exceptional character, in contrast with the formation of different salts and also different crystalline forms (i.e. polymorphs) of a substance whose is nowadays possible at least to predict the existence by means of proper software. This concept is indeed confirmed by the following actual observation: are known co-crystals of only 30% of the APIs containing carboxylic groups marketed nowadays in the United States.

The two co-crystals of Lapatinib monoacid salts, object of the present invention, according to our knowledge, appear not to be disclosed or exemplified in prior art documents. Vice-versa, a co-crystal of Lapatinib ditosylate with guanidine hydrochloride, i.e. a co-crystal of Lapatinib diacid salt with a conformer which is itself a salt (an hydrochloride), is disclosed in example 3 of WO2010/081443. Considering the structure or composition of said prior co-crystal of Lapatinib, the preparation of Lapatinib co-crystal in general and, specifically the preparation of co-crystals of Lapatinib monoacid salts, is an exceptional and not expected event, especially if it is considered that Lapatinib is known since many years and many studies directed to the preparation processes and to new solid forms have been carried out in the past years providing a lot of literature.

This is also confirmed by the fact that, although Lapatinib ditosylate is known to form numerous solvates, with solvents such as MeOH, IPA, NMP, dioxane, ACN, DMF, nitrobenzene and pyridine, all the experimental attempts to prepare co-crystals of Lapatinib ditosylated, using various co-formers, solvents and crystallization techniques, provided the only disclosure of example 3 of WO2010/081443.

The new discovered co-crystals of Lapatinib have the following composition:
(a) Lapatinib monotosylate : caproic acid (1:2),
(b) Lapatinib monotosylate : caprylic acid (1:2).

The special technical features that bounds these products is that they are co-crystals of Lapatinib monoacid salts, i.e. co-crystal formed by Lapatinib monoacid salt with a conformer, wherein the Lapatinib monoacid salt is Lapatinib monotosylate.

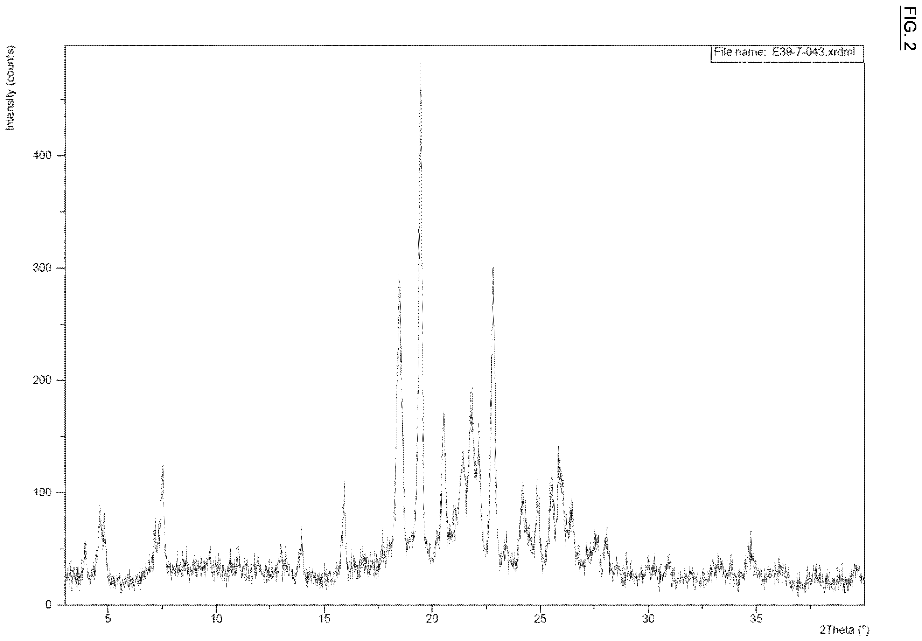

According to one embodiment, the co-crystal of Lapatinib being Lapatinib monotosylate : caproic acid (1:2) of formula (Form I) has a X-Ray Powder Diffractogram (XRPD) with characteristic peaks expressed in 2 Theta value of: 18,49, 19,47, 22,81 (+/-) 0,10 (See Fig. 2).

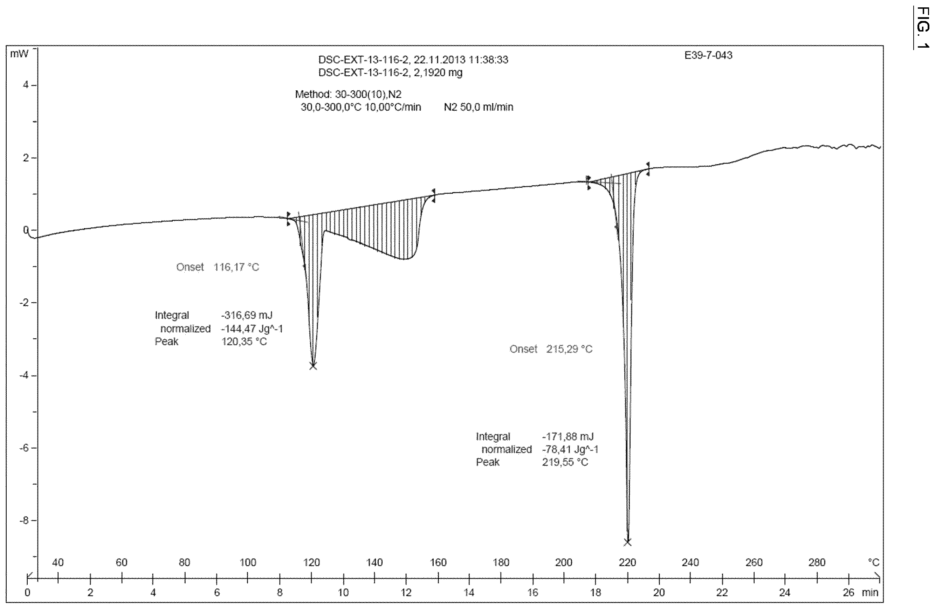

Co-crystal of Lapatinib being Lapatinib monotosylate : caproic acid (1:2) of formula (Form I) has an endothermic sharp peak with an onset at 116.17°C (See Fig. 1).

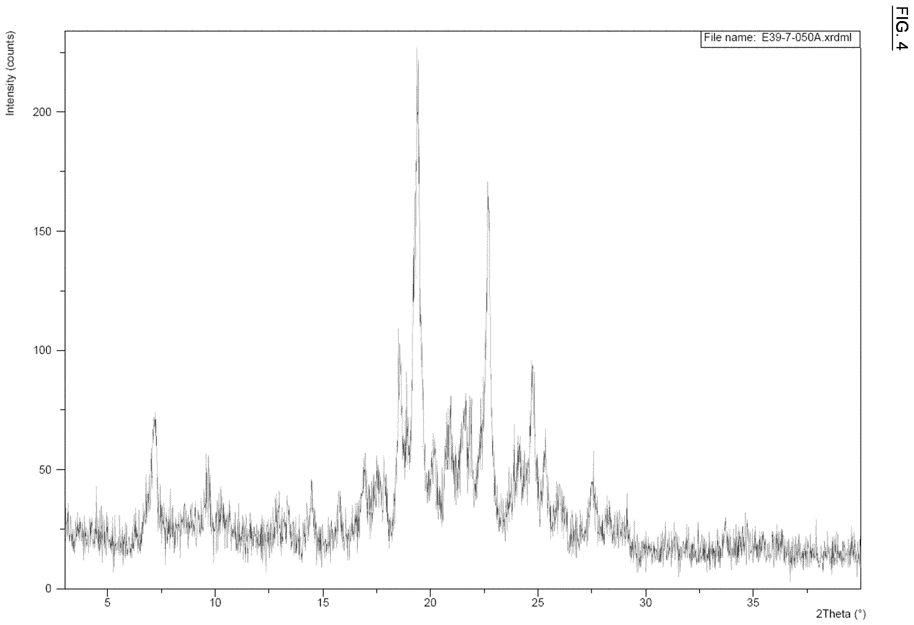

According to one embodiment, the co-crystal of Lapatinib being Lapatinib monotosylate : caprylic acid (1:2) of formula (Form II) has a X-Ray Powder Diffractogram (XRPD) with characteristic peaks expressed in 2 Theta value of: 18,55, 19,48, 22,71, 24,74 (+/-) 0,10 (See Fig. 4).

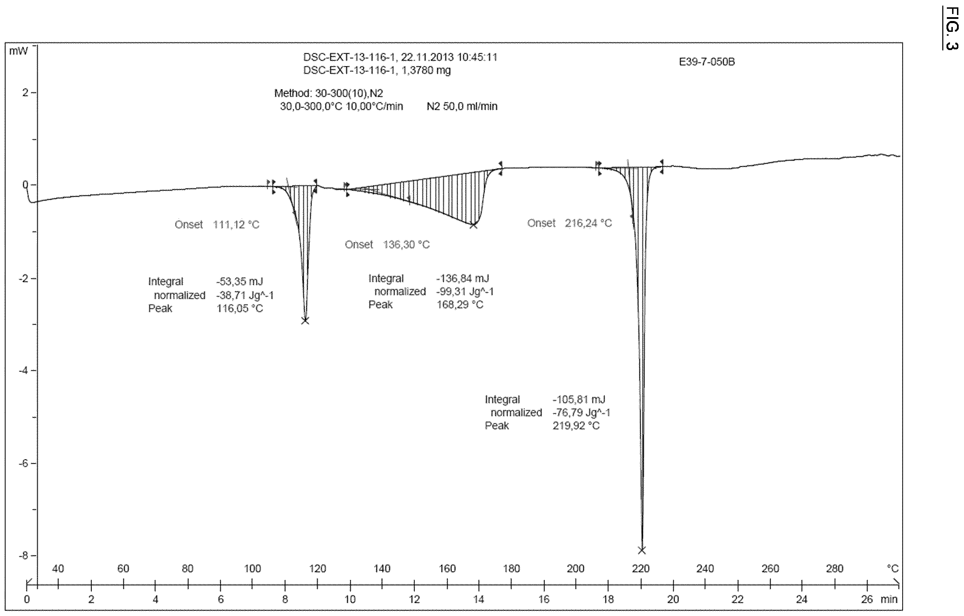

Co-crystal of Lapatinib being Lapatinib monotosylate : caprylic acid (1:2) of formula (Form II) has an endothermic sharp peak with an onset at 111.12°C (See Fig. 3).

As above said, the formation of a co-crystal is an accidental event since the obtainment of a structural arrangement of at least two molecules or salts thereof through weak forces like hydrogen bonding and π-π stacking, thus forming an ordinate aggregate, is unpredictable.

Proof of this unpredictable event is the stoichiometry of the co-crystals of Lapatinib monoacid salts of the present invention indeed the co-crystals of Lapatinib monotosylate comprise 2 molecules of Caproic acid or 2 molecules of Caprylic acid per one molecule of Lapatinib monotosylate. Such a stoichiometric ratio of Lapatinib monoacid salt versus the conformer is unexpected and unpredictable.

The know co-crystals of the active pharmaceutical ingredients typically comprises the active pharmaceutical ingredients as free acid or free base and one or more conformers. In the known co-crystal where the active pharmaceutical ingredient is in form as salt, typically said salts have not free acid or basic sites. In the specific case of Lapatinib, indeed, the known co-crystal consists in Lapatinib ditosylate (i.e. Lapatinib having not free basic sites) and guanidine hydrochloride as conformer.

Thus, the obtainment of a co-crystal of Lapatinib according to the present invention, wherein Lapatinib is in form of a monoacid salt has been a surprising event.

The formation of a cocrystal, and not a salt, of formula (Form 1) and (Form 2) is confirmed by the stoichiometry: 2 basic points for three acids (1 p-toluenesulfonic acid and 2 aliphatic acids).

As further confirmation that the products of the present inventions are co-crystals and not simply salts, the criterion adopted by the FDA Guidelines of April 2013 (see below) is here reported: the active ingredient-excipient complex should be classified as co-crystal if ΔpKa of the two species is <1. Although in the literature it has not been possible to find the pKa of the second less basic function of Lapatinib, however, by comparison with other related molecules (Gefitinib and Erlotinib), it is possible to approximate a pKa 5.4. The pKa of caproic acid is 4.8, pKa of caprylic acid is 4.91 and pKa of adipic acid is 4.4, so that for all the products the FDA's criterion to classify them as co-crystals is satisfied.

Co-crystals do not involve structural modification of the parent molecules, therefore, in the case of designing co-crystals of marketed drugs, their development programs (including clinical trials) shall be significantly shorter and less risky than those of New Chemical Entities (NCEs).

As a result of the increasing relevance of the co-crystals as solid forms, in April 2013, the FDA issued a guidance for industry regarding "Regulatory Classification of Pharmaceutical Co-Crystals". This regulation paves the way to the use of co-crystals of APIs for new chemical entities and generic products. Indeed, since co-crystals are defined as a drug product intermediate (dissociable "API-excipient" molecular complexes), this consideration opens a new path for generic companies to file ANDA avoiding the 505 (b)(2) regulatory track. This could be applied also to the Lapatinib co-crystals of the present inventions, with evident advantages.

Moreover, according to P. H. Stahl and C. G. Wermuth, editors, Handbook of Pharmaceutical Salts: Properties, Selection and Use, Weinheim/Zürich:Wiley-VCH/VHCA, 2002, the conformers of the three co-crystals of the present invention are pharmaceutically acceptable acids, so that the co-crystals of the present invention have the advantage to be suitable for pharmaceutical compositions and medical treatments.

Another aspect of the present invention is the process for the preparation of the co-crystal of Lapatinib being
(a) Lapatinib monotosylate : caproic acid (1:2) of formula (Form I): 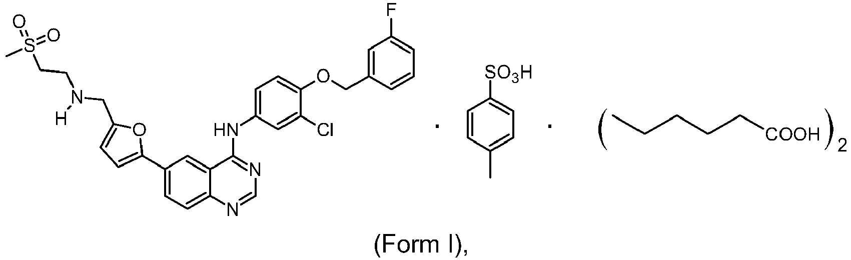 or being
(b) Lapatinib monotosylate : caprylic acid (1:2) of formula (Form II): 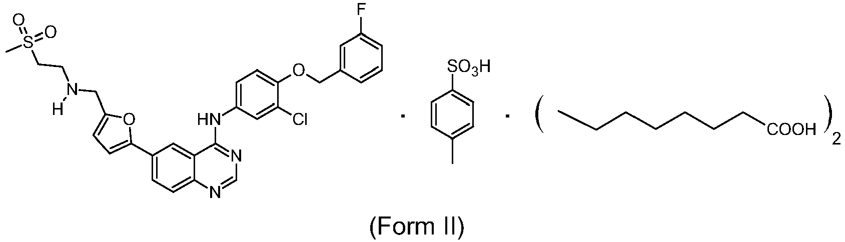 comprising the following steps:

(A.) reaction of Lapatinib of formula (I) with paratoluensulfonic acid to provide Lapatinib monotosylate of formula (I - TsOH): 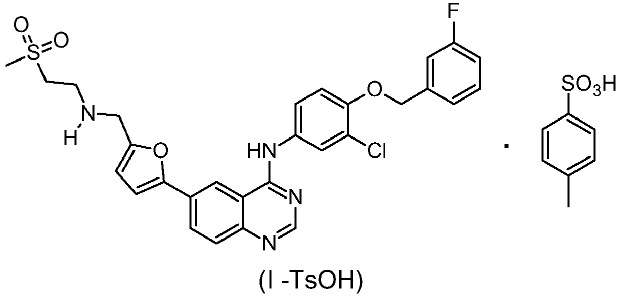
(B.) treatment of Lapatinib monotosylate respectively with caproic acid or caprylic acid.

Lapatinib monotosylate salt was prepared from Lapatinib free base following the procedure reported in WO2010027848A2 (Example 1).

The preliminary solubility tests showed that Lapatinib monotosylate is highly insoluble in all the assayed solvents (H₂O, MeOH, EtOH, IPA, ACN, acetone, MIK, EtOAc, iBuOAc, TBME, THF, CH₂Cl₂, toluene, cyclohexane), even after heating to reflux at a 40 mL·g⁻¹ concentration. As Lapatinib monotosylate is a yellow colored product, relative solubility could be qualitatively assessed by the colour of the mother liquors, being MeOH the solvent which offered the higher solubilization.

Two new co-crystals of Lapatinib monotosylate salts were obtained with caproic and caprylic acids with a ratio 1:2 (confirmed by NMR). Therefore, the formation of a co-crystal (of a salt) is confirmed by the stoichiometry: 2 basic points for three acids (1 p-toluenesulfonic acid and 2 aliphatic acids).

The two co-crystals obtained with caproic acid and caprylic acids were both obtained by slurrying in water and were scaled up from milligrams to 15 grams of Lapatinib monotosylate with excellent yield.

The method of preparation of co-crystal of formula (Form I) and (Form II) is scalable on industrial plant since:
- it is a method in solution (slurrying),
- it employs ICH guideline solvent class III (water),
- it provides high yield (from 86% to almost quantitative).

Despite their preparation in water, the co-crystals of the invention do not incorporate water molecules in the crystalline structure as demonstrated by TGA.

According to a preferred embodiment of the process for the preparation of the co-crystal of Lapatinib monotosylate : caproic acid (1:2) of formula (Form I), the amount of caproic acid is between 2.5 and 3.0 equivalents, being preferred 2.7 equivalents, since it provides the complete transformation of Lapatinib monotosylate to co-crystal of formula (Form I). Indeed, with 2.1 equivalents of caproic acid the transformation was not complete after one night. The excess of caproic acid was successfully eliminated with the washing, as confirmed by ¹H-NMR.

According to a preferred embodiment of the process for the preparation of the co-crystal of Lapatinib monotosylate : caprylic acid (1:2) of formula (Form II) the solvent mixture is composed by water : EtOH 5:1. Indeed, using a molar ratio between Lapatinib monotosylate and caplylic acid of 1 : 2.2, said mixture afforded the desired co-crystal with a complete conversion. EtOH was chosen instead of MeOH because both gave similar results and EtOH is a class 3 ICH guideline solvent.

According to a preferred embodiment of the process for the preparation of the co-crystal of Lapatinib monotosylate : caprylic acid (1:2) of formula (Form II) the isolated product is washed three times with 2.5 volumes of mixture water:EtOH 5:1, since performing only two washings, a very little residual caprylic acid was detected. A third washing afforded Form II without residual caprylic acid (controlled by ¹H-NMR).

According to a preferred embodiment of the process for the preparation of the co-crystal of Lapatinib monotosylate : caprylic acid (1:2) of formula (Form II) the amount of caprylic acid employed is comprised in the range from 2.2 to 2.5 equivalents, preferably 2.3 equivalents. Indeed, at 4 g scale, 2.3 equivalents of caprylic acid were required for complete transformation of Lapatinib monotosylate to co-crystal of formula (Form II).

According to a an aspect of the process for the preparation of the co-crystal of Lapatinib monotosylate : caprylic acid (1:2) of formula (Form II) the experiment at 13.5 g scale had to be reprocessed because the solid obtained was not homogeneous (the diffraction of some samples showed complete transformation into Form II but others showed residual crystalline Lapatinib monotosulate). After reprocessing the solid with 1 extra equivalent of caprylic acid the solid had to be resuspended in 20 volumes of mixture water: EtOH, 5:1.

The preparation of the co-crystal having formula (Form II), at 13.5 g scale, includes two steps:
- formation of co-crystal of formula Form II with a small excess of Lapatinib monotosylate,
- reprocessing of the co-crystal to ensure complete conversion. (See examples 15 and 16).

In conclusion, increasing the scale of the experiments of the process for the preparation of the co-crystals of formula (Form I) and (Form II), some little modifications had to be made in order to achieve complete transformation of the starting products and to eliminate completely the excess of the coformers:
- in co-crystals of formula (Form I) and (Form II) a larger amount of coformer was required (2.3 or better 2.5 or best 2.7 equivalents instead of 2.1 equivalents).
- In Forms II additional washings (three instead of two) were needed in order to eliminate the excess of the coformer.

In conclusion, co-crystals of Lapatinib of formula (Form I) and (Form II) are prepared using Lapatinib monotosylate as starting product. These two co-crystals were obtained by slurrying with very good yields at mg to multigrams scale.

The method of slurrying for the preparation of said co-crystals can be difficult to scale-up because the conversion rate depends highly on the stirring rate and stirring type, which can be very different at small and higher scale. Therefore, the different parameters of the slurrying (stirring, dilution, amount of coformers...) needed be optimized for each scale-up.

With the increase of the scale, a higher amount of coformers was necessary in order to obtain complete conversion. This problem of conversion could be due to the use of a mechanical stirring (no attrition effect as for magnetical stirring) or due to the formation of dense mixtures worsening the homogenization.

The use of higher amounts of coformer led to a problem of elimination of this excess and additional washings had to be carried out to obtain the pure crystal form. An additional suspension could to be performed in the case of Form II to eliminate the large excess of caprylic acid after the reprocessing step.

Co-crystal of Lapatinib of formula (Form I) was prepared from Lapatinib monotosylate by slurrying in water in the presence of 2.7 eq. of caproic acid. 95% yield.

Co-crystal of Lapatinib of formula (Form II) was prepared from Lapatinib monotosylate by slurrying in water/EtOH (5:1) in the presence of 2.3 eq. of caprylic acid followed by a reprocessing in water/EtOH (5:1) in the presence of 1 eq. of caprylic acid. 86% overall yield.

According to the processes above described, the salts of Lapatinib with a monoprotic acid in stoichiometry (1:1) can be used for the preparation of co-crystals of Lapatinib. The monoprotic acid can be e.g. HCl, HBr, Benzensolfonic acid, p-Toluensofonic acid, formic acid, acetic acid, propionic acid, butirric acid, etc.

In particular, the salt Lapatinib monotosylate is useful for the preparation of co-crystals of Lapatinib.

More preferably, salts of Lapatinib with a monoprotic acid in stoichiometry (1:1), and in particular Lapatinib monotosylate, can be used for the preparation of co-crystals of Lapatinib and, in particular, for the preparation of the two co-crystals having formula (Form I), (Form II).

The co-crystals of Lapatinib of the present invention having formula (Form I), (Form II) are useful in medicine.

In particular, the co-crystal of Lapatinib of the present invention can be used in a method for treatment of breast cancer and other solid tumours.

More in particular, the co-crystal of Lapatinib of the present invention can be used for the treatment of patients with advanced or metastatic breast cancer whose tumors overexpress HER2 (ErbB2).

To employ the co-crystals of Lapatinib of the present invention for medical uses it is necessary to prepare pharmaceutical compositions comprising said a co-crystal of Lapatinib with one or more excipients, carriers or diluents pharmaceutically acceptable.

The pharmaceutical compositions comprising the co-crystals of Lapatinib of the present invention can be orally administrated.

The pharmaceutical compositions comprising the co-crystals of Lapatinib of the present invention can be in the form of film-coated tablets and can comprise an amount of co-crystal equivalent to from 50 to 300 mg Lapatinib free base, preferably 250 mg.

The pharmaceutical compositions comprising the co-crystals of Lapatinib of the present invention can further comprise one or more of the following inactive ingredients: cellulose - microcrystalline, povidone (K30), croscarmellose sodium, sodium starch glycolate and magnesium stearate.

The stabilities studies of co-crystals of Lapatinib of the present invention shown that said co-crystals are stable. Indeed, Forms I, II remain stable for at least 115 days at room temperature and 94% RH.

The conclusions of the stability studies at controlled humidity and temperature conditions are: the co-crystals of Lapatinib of the present inventions are stable (see example 19), thus suitable for pharmaceutical and medical uses.

Furthermore, the ranking of stability at 40°C with 74% RH is the following: co-crystal of formula (Form III) > co-crystal of formula (Form II) > co-crystal of formula (Form I), thus the co-crystal of formula (Form III) is the one more stable. The Form III indeed remained stable after 97 days at 40 °C - 74% RH.

Co-crystal Form III (Lapatinib·HCl·Adipic acid (1:1)) is the form offering highest solubility. Form III is about 4 times more soluble than Lapatinib ditosylate.

Furthermore, the ranking of solubility is the following: co-crystal of formula (Form III) > co-crystal of formula (Form I) > co-crystal of formula (Form II) ca. = Lapitinib ditosylate.

Co-crystal Form III (Lapatinib·HCl·Adipic acid (1:1)) is the form offering highest stability and solubility.

Co-crystals of Lapatinib tosylate indicated that Form I is more soluble than Form II (see example 20). However, the lower solubility of Form II seems to increase the stability of the crystalline form in humidity conditions (40°C, 74% RH and water slurrying).

A pharmacokinetic and bioequivalence study has been carried out to determine the pharmacokinetic profile and the bioavailability of Lapatinib when administered orally as a ditosylate monohydrate (form currently on the market; comparative reference product) or as three different solid co-crystal formulations to male Wistar rats at 30 mg Lapatinib/kg (See example 21). Hence, the pharmacokinetic behavior of Lapatinib was also assessed following its intravenous bolus administration at a dose level of 10 mg Lapatinib/kg.

For the intravenous administration, a solution of Lapatinib ditosylate monohydrate was prepared in DMSO:PEG:WFI (10:40:50) to reach a concentration of 2.5 mg parent compound/mL, wherein DMSO, PEG and WFI mean respectively dimethylsulphoxide, polyethyleneglycol and water for injection.

For the oral administration, the compounds were suspended in hydroxypropylmethylcellulose:Tween 80 (0.5%:0.1%) in WFI to reach a concentration of 3 mg parent compound/mL in all cases.

Blood samples (three animals per time point) were obtained from the retro-orbital plexus of the animals at pre-dose, 2 min (only iv), 5 min (only po) and 15 min, 30 min, 1 h, 2 h, 3 h, 8 h and 24 h after administration.

A bioanalytical UPLC^{®}-MS/MS method was characterized and allowed the determination of the Lapatinib in rat plasma; the precision and accuracy of the QC samples was appropriate according to international guidelines.

A non-compartmental pharmacokinetic analysis was carried out with mean plasma concentration-time curves using WinNonlin® Professional Version 6.3 (Phoenix Version 1.3, Pharsight Corporation):

The following conclusions could be drawn from the results obtained:

A) Following an intravenous bolus administration at 10 mg/kg, mean plasma levels declined as a function of time showing two disposition phases (from the first sampling time up to 3 hours postdose, and from 8 to 24 hours postadministration). However, the fact that only two time points corresponding to this second phase (terminal elimination phase) were obtained did not allow the calculations of the pharmacokinetic parameters derived from this terminal phase (AUC_{inf}, t_{1/2}, MRT, V_{z}, Vₛₛ and CL).

B) Values of 20222 ng/mL and 13661 ng·h/mL were obtained for the extrapolated concentration at time 0 (Co) and for AUCₜ, respectively.

C) The plasma profiles obtained for Lapatinib after its oral administration as a ditosylate monohydrate (comparative reference form) or as three different solid crystal formulations were similar. There was a rapid absorption phase (peak plasma levels reached at 2 or 3 hours postdose) followed by fast decay.

D) The highest peak plasma Lapatinib levels were reached for the Lapatinib ditosylate monohydrate and the co-crystal Lapatinib monotosylate: caproic acid 1:2.

E) The highest AUC values were obtained for the Lapatinib ditosylate monohydrate (referencel form) and the co-crystal Lapatinib monotosylate: caprylic acid 1:2. Consequently, the comparison of AUCₜ values and intravenous administration resulted in a higher absolute bioavailability for these two formulations (57.49 and 50.70%, respectively) than for the other crystal formulations (32.42 and 44.98%).

Furthermore, Lapatinib ditosylate monohydrate and the co-crystal Lapatinib monotosylate: caprylic acid 1:2, considering the confidence interval, have substantially the same bioavailability.

F) Values of the apparent absorption rate (Cₘₐₓ/AUCₜ) were similar for all the compounds (ranging from 0.135 to 0.149 h⁻¹). Nevertheless the co-crystal Lapatinib monotosylate: caproic acid 1:2 (Form 1) showed a higher value (0.239 h⁻¹) (even in comparison with Lapatinib ditosylate monohydrate), which indicated a faster absorption of Lapatinib when Lapatinib is in such a solid form. Therefore, co-crystal Lapatinib monotosylate: caproic acid 1:2 is suitable for immediate-release or fast-release composition of Lapatinib.

G) From all the tested formulations, the Lapatinib ditosylate monohydrate and the co-crystal Lapatinib monotosylate: caprylic acid 1:2 seemed to be the most favorable in terms of absolute bioavailability. The elimination of Lapatinib from these two formulations seemed slower than from the rest of crystals since lower CL/F and longer half-lives were obtained.

H) Finally, apparent elimination half-life and mean residence time values obtained for the co-crystal Lapatinib monotosylate: caprylic acid 1:2 (2.62 and 4.67 h, respectively) were slightly longer than those obtained for the ditosylate monohydrate (2.39 and 4.28 h, respectively).

The co-crystals of the present invention, being co-crystal of Lapatinib monoacid salts selected in the group consisting of:
(a) Lapatinib monotosylate : caproic acid (1:2) of formula (I),
(b) Lapatinib monotosylate : caprylic acid (1:2) of formula (Form II);
are suitable for medical uses, in particular to treat cancers and tumours, preferably for oral administration.

The co-crystal Lapatinib monotosylate : caprylic acid (1:2) of formula (Form II) is preferred since it provide substantially the same bioavailability of the Lapatinib ditosylate monohydrate. It should be considered that the choice of the salt Lapatinib ditosylate monohydrate has been justified based on crystalline properties and good bioavailability (see Tyverb : EPAR - Public assessment report). Since co-crystal Lapatinib
monotosylate : caprylic acid (1:2) provides substantially the same bioavailabilty of a compound practically insoluble in aqueous media, the Lapatinib ditosylate monohydrate, which has previously been selected over many other solid forms of Lapatinib considering his good bioavailability, it appears that the co-crystal Lapatinib monotosylate : caprylic acid (1:2) is one of the best compound of Lapatinib in terms of bioavailablity.

The co-crystal Lapatinib monotosylate : caproic acid (1:2) of formula (I) is suitable for immediate or fast release pharmaceutical compositions of Lapatinib.

All the two co-crystals of Lapatinib of the present invention provides much higher plasmatic concentration after 5 minutes by the oral administration in comparison with the reference compound Lapatinib ditosylate monohydrate (See table 3 of example 21).

Thus, an object of the present invention is an immediate release pharmaceutical composition comprising a co-crystal of Lapatinib monoacid salts selected in the group consisting of:
(a) Lapatinib monotosylate : caproic acid (1:2) of formula (Form I),
(b) Lapatinib monotosylate : caprylic acid (1:2) of formula (Form II);
and one or more pharmaceutically acceptable excipients.
An example of an immediate release pharmaceutical composition comprising a co-crystal of Lapatinib monoacid comprises a co-crystal of Lapatinib of formula (Form I) or (Form II) in hydroxypropylcellulose : Tween 80 (0.5% : 0.1 %) in water for injection. Therefore, the co-crystal of Lapatinib monoacid salts of the present invention can be used for immediate release oral pharmaceutical form or for oral immediate release dosage forms.

### EXPERIMENTAL PART

### Example 1 - Preparation of Lapatinib monotosylate of formula (I, 1 TsOH).

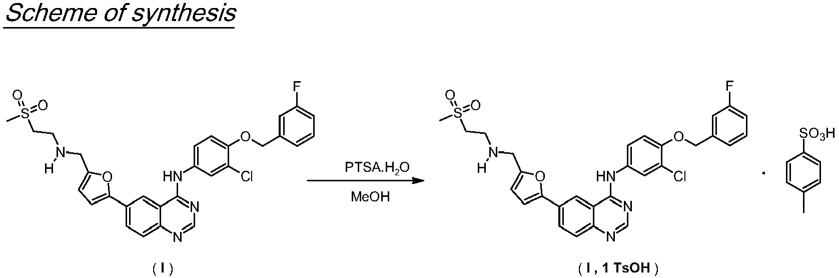

Lapatinib free base of formula (I) (2.00 g, 3.44 mmol) was suspended in MeOH (80 mL, 40 mL·g⁻¹). To the brown/yellow suspension, PTSA·H₂O (0.67 g, 3.52 mmol, 1.02 eq) was added and the resulting bright yellow mixture was stirred at r.t. overnight. The solid was filtered, washed with MeOH (5 mL, 2.5 mL·g⁻¹) and dried under vacuum at r.t., affording Lapatinib monotosylate of formula (I, 1 TsOH) (2.46 g, 3.27 mmol, 95% molar yield) as a yellow solid.

### Characterization:

¹H-NMR (*d₆*-DMSO, 400 MHz): δ (ppm) = 9.99 (s, 1H), 8.85 (s, 1H), 8.60 (s, 1H), 8.23 (dd, J = 8.8, 1.6, 1H), 7.99 (d, J = 2.4, 1H), 7.86 (d, J = 8.8, 1H), 7.73 (dd, J = 8.8, 2.4, 1H), 7.52-7.44 (m, 3H), 7.37-7.26 (m, 3H), 7.23-7.14 (m, 1H), 7.10 (d, J = 7.6, 2H), 6.84 (d, J = 2.4, 1H), 5.27 (s, 2H), 4.40 (s, 2H), 3.61-3.49 (m, 2H), 3.47-3.39 (m, 2H), 3.19-3.10 (m, 3H), 2.28 (s, 3H).

XRPD: 2theta (⊖) = 5.2, 6.0, 7.4, 8.6, 9.1, 10.0, 12.7, 14.8, 15.7, 17.6, 18.6, 19.8, 21.5, 22.2, 23.3, 23.9.

### Example 2 - Preparation of co-crystal Lapatinib monotosylate : caproic acid (1:2) of formula (Form I):

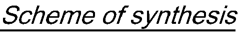

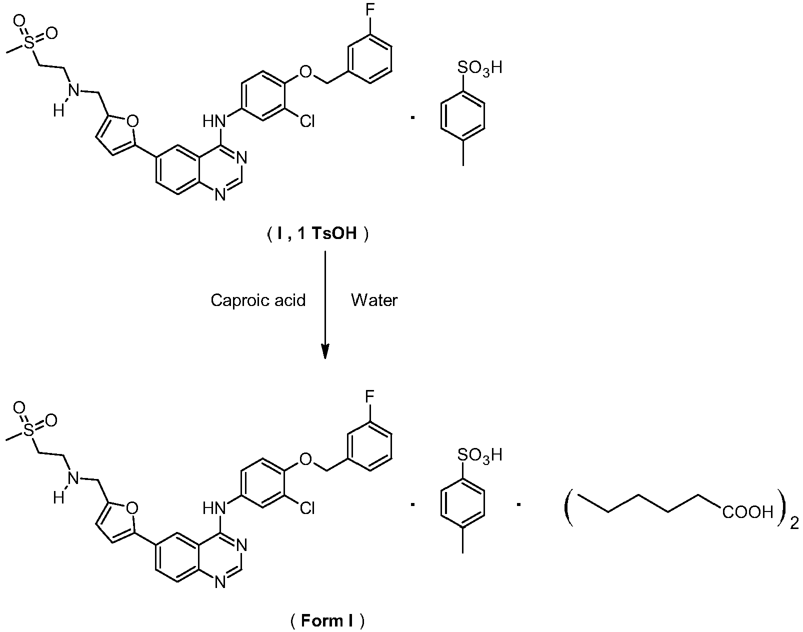

Lapatinib monotosylate of formula (I, 1 TsOH) as prepared in example 1 (0.20 g, 0.266 mmol) was suspended in H₂O (4 mL, 20 mL·g-1). Caproic acid (0.07 mL, 0.559 mmol, 2.1 eq.) was added and the resulting suspension was stirred at r.t. overnight. The solid was filtered, washed with water and dried under vacuum at r.t., affording the desired cocrystal (0.27 g, quantitative yield) as an off-white solid.

Form I obtained by slurrying in water was characterized by several techniques:
- ¹H-NMR: Proton nuclear magnetic resonance analyses were recorded in deuterated dimethyl sulfoxide (DMSO-*d₆*) in a Varian Mercury 400 spectrometer, equipped with a broadband probe ATB 1 H/19F/X of 5 mm. Spectra were acquired dissolving 5-10 mg of sample in 0.6 mL of deuterated solvent.

¹H-NMR (*d₆*-DMSO, 400 MHz): δ (ppm) = 11.72 (bs, 1H), 9.98 (s, 1H), 9.31 (bs, 1H), 8.84 (s, 1H), 8.60 (s, 1H), 8.22 (dd, J = 8.8, 1.6, 1H), 7.98 (d, J = 2.4, 1H), 7.86 (d, J = 8.8, 1H), 7.72 (dd, J = 8.8, 2.4, 1H), 7.50-7.44 (m, 3H), 7.34-7.27 (m, 3H), 7.20-7.14 (m, 2H), 7.09 (d, J = 8.0, 2H), 6.83 (d, J = 3.2, 1H), 5.26 (s, 2H), 4.39 (s, 2H), 3.56-3.52 (m, 2H), 3.44-3.40 (m, 2H), 3.12 (s, 3H), 2.28 (s, 3H), 2.18 (t, J = 7.2, 4H), 1.55-1.44 (m, 4H), 1.33-1.19 (m, 8H), 0.86 (t, J = 6.8, 6H).

- DSC: DSC analysis was recorded with a Mettler DSC822^{e}. A sample of 2.1920 mg was weighed into a 40 µL aluminium crucible with a pinhole lid and was heated, under nitrogen (50 mL/min), at 10°C/min from 30 to 300°C.

Form I is characterized by an endothermic sharp peak with an onset at 116.17 °C measured by DSC analysis (10 °C/min). Visual observation of the crystal did not show a melting: therefore it could be a solid-solid transition or a melting with an instantaneous recrystallization to the monotosylate salt.

Subsequently, a broad endothermic peak that could be due to caproic acid evaporation was observed (enthalpy fusion and evaporation -144.47 J/g). Another endothermic sharp peak corresponding to the melting of Lapatinib monotosylate salt with an onset at 215.29 °C (enthalpy fusion -78.41 J/g) was observed. (See Figure 1 - DSC).

- TGA: Thermogravimetric analysis was recorded in a thermogravimetric analyzer Mettler TGA/SDTA851^{e}. A sample of 7.4451 mg was weighed into a 70 µL alumina crucible with a pinhole lid and was heated at 10 °C/min from 30 to 300 °C, under nitrogen (50 mL/min). The TG analysis of Form I shows a 23.31% weight loss starting partially before the melting point (between 75 °C and 185 °C). This loss of weight could come from the evaporation of caproic acid (23.31% loss - calculated loss for 2 caproic acid molecules 23.57%).

- XRPD: XRPD analysis was performed using a PANalytical X'Pert diffractometer with Cu Kα radiation in Bragg-Brentano geometry. The system is equipped with a monodimensional, real time multiple strip detector. The diffractogram was recorded from 3° to 40° (2θ) at a scan rate of 17.6° per minute (see Figure 2). List of selected peaks (only peaks with relative intensity greater than or equal to 1% are indicated):

| Pos. [°2Th] | d-spacing [Å] | Rel. Int. [%] |
|---|---|---|
| 3,96 | 22,33 | 6 |
| 4,63 | 19,08 | 14 |
| 7,16 | 12,34 | 9 |
| 7,54 | 11,73 | 19 |
| 11,04 | 8,01 | 3 |
| 13,10 | 6,76 | 2 |
| 13,95 | 6,35 | 8 |
| 15,93 | 5,56 | 16 |
| 18,49 | 4,80 | 58 |
| 19,47 | 4,56 | 100 |
| 20,54 | 4,32 | 33 |
| 21,41 | 4,15 | 24 |
| 21,76 | 4,08 | 33 |
| 22,18 | 4,01 | 25 |
| 22,81 | 3,90 | 61 |
| 24,19 | 3,68 | 18 |
| 24,85 | 3,58 | 18 |
| 25,54 | 3,49 | 20 |
| 25,85 | 3,45 | 23 |
| 26,48 | 3,37 | 14 |
| 27,63 | 3,23 | 9 |
| 28,05 | 3,18 | 8 |
| 30,12 | 2,97 | 2 |
| 30,95 | 2,89 | 4 |
| 33,09 | 2,71 | 3 |
| 33,82 | 2,65 | 4 |
| 34,69 | 2,59 | 7 |
| 36,28 | 2,48 | 3 |
| 37,81 | 2,38 | 2 |

The co-crystal of Lapatinib being Lapatinib monotosylate : caproic acid (1:2) of formula (Form I) has a X-Ray Powder Diffractogram (XRPD) with characteristic peaks expressed in 2 Theta value of: 4,63 (m), 7,54 (m), 15,93 (m), 18,49 (s), 19,47 (vs), 20,54 (m), 21,41 (m), 21,76 (m), 22,18 (m), 22,81 (s), 24,19 (m), 24,85 (m), 25,54 (m), 25,85 (m), 26,48 (m), (+/-) 0,10; wherein (vs) = very strong intensity, (s) = strong intensity, (m) = medium intensity. In particular, the co-crystal having formula (Form I) has characteristic peaks expressed in 2 Theta value of: 18,49, 19,47, 22,81 (+/-) 0,10. (See Figure 2 - XRPD Diffractogram).

### Example 3 - Preparation of co-crystal Lapatinib monotosylate : caprylic acid (1:2) of formula (Form II):

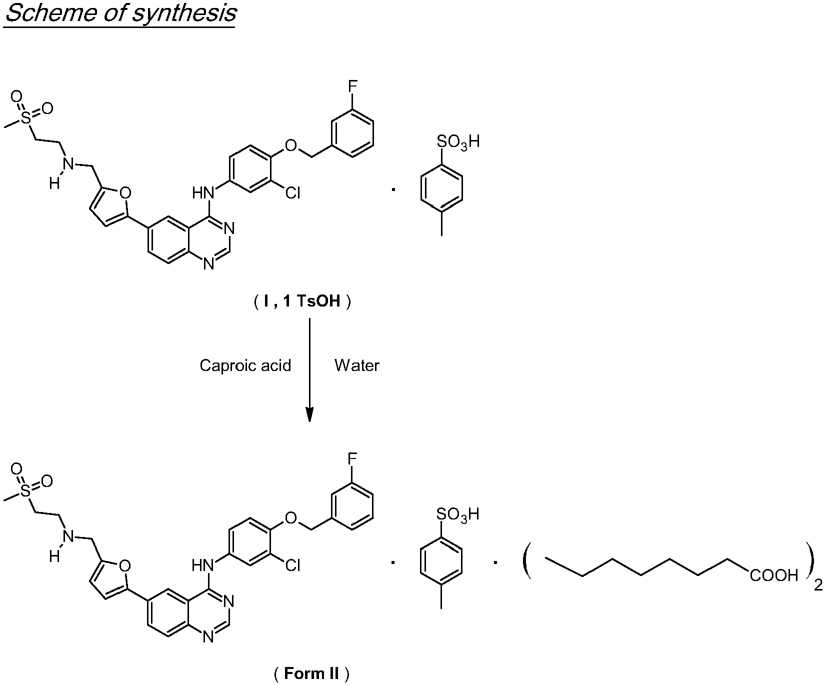

Lapatinib monotosylate (0.20 g, 0.266 mmol) was suspended in H₂O (4 mL, 20 mL·g-1). Caprylic acid (83 mg, 0.576 mmol, 2.1 eq) was added and the resulting suspension was stirred at r.t. overnight. The solid was filtered, washed with water and dried under vacuum at r.t., affording the desired co-crystal (0.28 g, quantitative yield) as an off-white solid.

Similar results were obtained using a H₂O:MeOH 3:1 mixture as solvent.

Co-crystal of formula (Form II) obtained by slurrying in water was characterized by several techniques:
- ¹H-NMR: Proton nuclear magnetic resonance analyses were recorded in deuterated dimethyl sulfoxide (DMSO-*d₆*) in a Varian Mercury 400 spectrometer, equipped with a broadband probe ATB 1 H/19F/X of 5 mm. Spectra were acquired dissolving 5-10 mg of sample in 0.6 mL of deuterated solvent.

¹H-NMR (*d₆*-DMSO, 400 MHz): δ (ppm) = 11.74 (bs, 1H), 9.98 (s, 1H), 8.85 (s, 1H), 8.60 (s, 1H), 8.23 (dd, J = 8.8, 1.6, 1H), 7.99 (d, J = 2.4, 1H), 7.87 (d, J = 8.8, 1H), 7.73 (dd, J = 8.8, 2.4, 1H), 7.53-7.43 (m, 3H), 7.37-7.26 (m, 3H), 7.23-7.14 (m, 2H), 7.10 (d, J = 8.0, 2H), 6.84 (d, J = 3.2, 1H), 5.27 (s, 2H), 4.40 (s, 2H), 3.57-3.52 (m, 2H), 3.47-3.40 (m, 2H), 3.13 (s, 3H), 2.28 (s, 3H), 2.18 (t, J = 7.2, 4H), 1.52-1.43 (m, 4H), 1.33-1.19 (m, 12H), 0.85 (t, J = 6.8, 6H).

- DSC: DSC analysis was recorded with a Mettler DSC822e. A sample of 1.3780 mg was weighed into a 40 µL aluminium crucible with a pinhole lid and was heated, under nitrogen (50 mL/min), at 10 °C/min from 30 to 300 °C. A similar DSC profile to form I was observed. Form II is characterized by an endothermic sharp peak with an onset at 111.12°C (enthalpy fusion -38.71 J/g) measured by DSC analysis (10 °C/min) (see Figure 3). Visual observation of the crystal did not show a melting: therefore it could be a solid-solid transition or a melting with an instantaneous recrystallization to the monotosylate salt. Subsequently, a broad endothermic peak that could be due to caprylic acid evaporation was observed (enthalpy evaporation - 99.31 J/g). Another endothermic sharp peak corresponding to the melting of Lapatinib monotosylate salt with an onset at 216.24 °C (enthalpy fusion -76.79 J/g) was observed (see figure 3).

- TGA: Thermogravimetric analysis was recorded in a thermogravimetric analyzer Mettler TGA/SDTA851^{e}. A sample of 5.6640 mg was weighed into a 70 µL alumina crucible with a pinhole lid and was heated at 10°C/min from 30 to 300°C, under nitrogen (50 mL/min). The TG analysis of Form I shows a 27.37% loss weight starting partially before the melting point (between 91 °C and 195 °C). This loss of weight could come from the evaporation of caprylic acid (27.68% loss - calculated loss for 2 caproic acid molecules 27.68%).

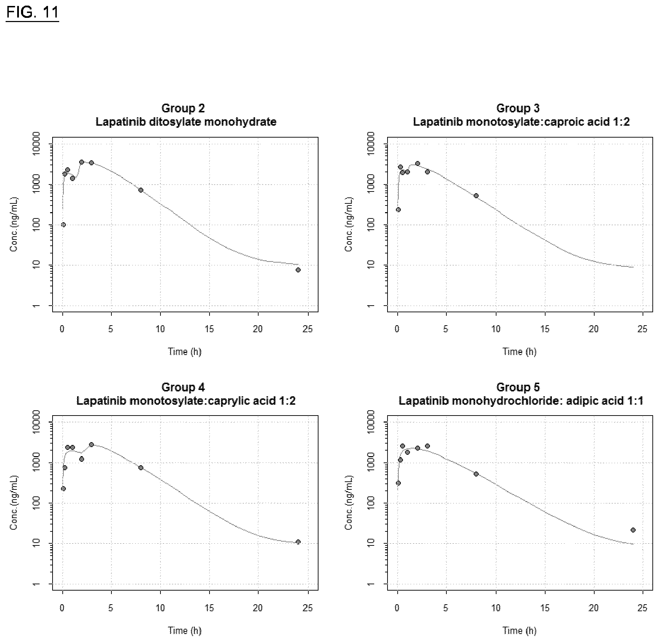

- XRPD: XRPD analysis was performed using a PANalytical X'Pert diffractometer with Cu Kα radiation in Bragg-Brentano geometry. The system is equipped with a monodimensional, real time multiple strip detector. The diffractogram was recorded from 3° to 40° (2θ) at a scan rate of 17.6° per minute (see Figure 11). List of selected peaks (only peaks with relative intensity greater than or equal to 1 % are indicated):

| Pos. [°2Th] | d-spacin g [Å] | Rel. Int. [%] |
|---|---|---|
| 7,25 | 12,20 | 30 |
| 9,74 | 9,08 | 13 |
| 13,16 | 6,73 | 4 |
| 14,49 | 6,11 | 12 |
| 15,78 | 5,62 | 10 |
| 16,91 | 5,24 | 18 |
| 17,76 | 4,99 | 13 |
| 18,55 | 4,78 | 41 |
| 19,48 | 4,56 | 100 |
| 20,16 | 4,41 | 25 |
| 20,93 | 4,24 | 32 |
| 21,58 | 4,12 | 33 |
| 21,88 | 4,06 | 31 |
| 22,71 | 3,92 | 89 |
| 24,06 | 3,70 | 21 |
| 24,74 | 3,60 | 46 |
| 25,34 | 3,52 | 25 |
| 26,08 | 3,42 | 12 |
| 27,52 | 3,24 | 18 |
| 28,95 | 3,08 | 6 |

The co-crystal of Lapatinib being Lapatinib monotosylate : caprylic acid (1:2) of formula (Form II) has a X-Ray Powder Diffractogram (XRPD) with characteristic peaks expressed in 2 Theta value of: 7,25 (m), 9,74 (m), 14,49 (m), 15,78 (m), 16,91 (m), 17,76 (m), 18,55 (s), 19,48 (vs), 20,16 (m), 20,93 (m), 21,58 (m), 21,88 (m), 22,71 (s), 24,06 (m), 24,74 (s), 25,34 (m), 26,08 (m), 27,52 (m), (+/-) 0,10; wherein (vs) = very strong intensity, (s) = strong intensity, (m) = medium intensity. In particular, the co-crystal having formula (Form II) has characteristic peaks expressed in 2 Theta value of: 18,55, 19,48, 22,71, 24,74 (+/-) 0,10. (See Figure 4 - XRPD Diffractogram).

XRPD comparison between forms I and II indicates that the crystalline forms are similar. However, this could be expected because the coformers are similar and the ratio between Lapatinib monotosylate salt and the coformers is identical (1:2).

### Example 4 - Preparation of Lapatinib monohydrochloride of formula (I, 1 HCl).

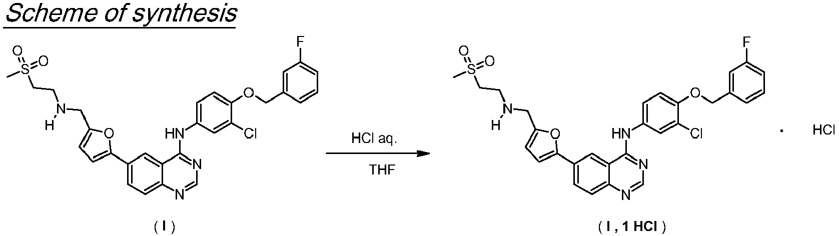

Lapatinib free base of formula (I) (1.00 g, 1.72 mmol)was suspended in THF (20 mL, 20 mL·g-1). To the brown/yellow suspension, HCl 1 N (1.70 mL 1.70 mmol, 1.0 eq) was added and the resulting bright yellow mixture was stirred at r.t. overnight. The solid was filtered, washed with THF and dried under vacuum at r.t., affording Lapatinib monohydrochloride of formula (I, 1 HCl) (1.05 g, 1.70 mmol, 95% yield).

### Characterization of Lapatinib monohydrochloride salt:

¹H-NMR: (*d₆*-DMSO, 400 MHz): δ (ppm) = 10.19 (s, 1H), 9.69 (bs, 1H), 9.20 (s, 1H), 8.61 (s, 1H), 8.23 (dd, J = 8.8, 1.6, 1 H), 8.14 (d, J = 2.4, 1H), 7.89-7.83 (m, 2H), 7.50-7.44 (m, 1H), 7.34-7.27 (m, 3H), 7.19-7.15 (m, 2H), 6.80 (d, J = 3.6, 1H), 5.26 (s, 2H), 4.40 (s, 2H), 3.66-3.62 (m, 2H), 3.44-3.40 (m, 2H), 3.13 (m, 3H).

XRPD: Analysis of the monohydrochloride salt by XRPD indicates a form with a moderate crystallinity having peaks at 2theta values of 5,0, 7,5, 8,2, 10,0, 10,8, 11,7, 15,1, 15,5, 17,6, 20,3, 21,7.

### Example 5 - Preparation of Lapatinib dihydrochloride of formula (I, 2 HCl).

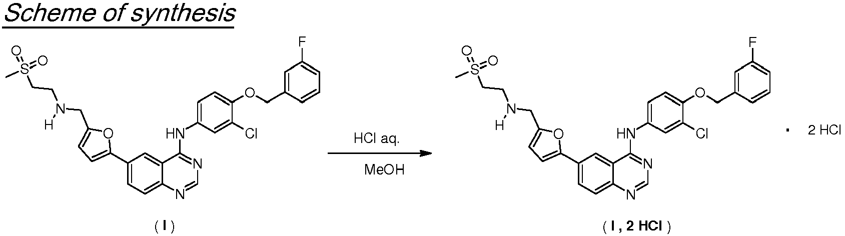

The Lapatinib dihydrochloride salt was prepared following the procedure reported in WO2010027848A2 (Example 6):
Lapatinib free base (1.01 g, 1.74 mmol) was suspended in MeOH (30 mL, 30 mL·g⁻¹). To the brown/yellow suspension, HCl 37% (0.33 mL, 3.9 mmol, 2.2 eq) was added and the resulting bright yellow mixture was stirred at r.t. overnight. The solid was filtered, and dried under vacuum at r.t., affording Lapatinib dihydrochloride of formula (I, 2 HCl) (1.06 g, 1.62 mmol, 93% yield). The diffractogram matched with the XRPD described in the application WO2010027848A2 for form C1 - Fig 5.1. and 5.2.

Comparison of ¹H-NMR and XRPD of the Lapatinib monohydrochloride salt prepared in example 4 with the Lapatinib dihydrochloride salt prepared in example 5 and with the Lapatinib free base confirms that Lapatinib monohydrochloride salt was effectively formed and it is not contaminated by the Lapatinib dihydrochloride or by the Lapatinib free base.

### Example 6 - Preparation of co-crystal Lapatinib monohydrochloride : adipic acid (1:1) of formula (Form III), which is not part of the present invention:

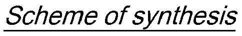

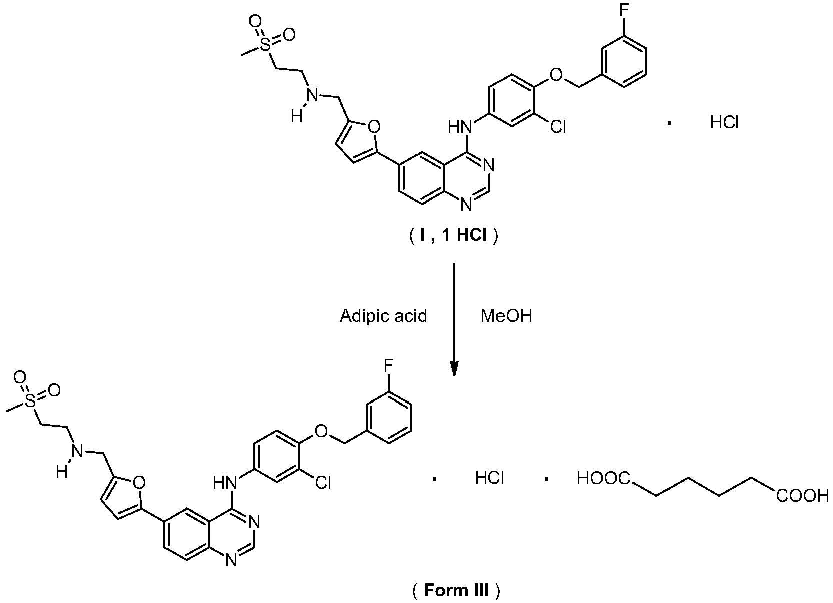

Lapatinib monohydrochloride (100 mg, 162 µmol) and adipic acid (50 mg, 342 µmol, 2.1 eq) were suspended in MeOH (2.0 mL). The resulting suspension (from yellow to off-white) was optionally seeded with previously obtained form III (A: seeded and C: without seeding) and the mixture was stirred overnight at r.t. The solid was filtered, washed with MeOH (0.4 mL) and dried under vacuum, affording in both cases the form III (105 mg, 85% yield) as a pale yellow solid.

### Co-crystal of formula (Form III) obtained by slurrying in MeOH was characterized by several techniques:

- ¹H-NMR: Proton nuclear magnetic resonance analyses were recorded in deuterated dimethyl sulfoxide (DMSO-d6) in a Varian Mercury 400 spectrometer, equipped with a broadband probe ATB 1 H/19F/X of 5 mm. Spectra were acquired dissolving 5-10 mg of sample in 0.6 mL of deuterated solvent.

¹H-NMR (*d₆*-DMSO, 400 MHz): δ (ppm) = 10.15 (bs, 1H), 9.16 (s, 1H), 8.61 (s, 1H), 8.23 (dd, J = 8.4, 1.6, 1H), 8.13 (d, J = 2.3, 1H), 7.93-7.81 (m, 2H), 7.52-7.43 (m, 1H), 7.37-7.26 (m, 3H), 7.22-7.15 (m, 2H), 6.81 (d, J = 3.5, 1H), 5.27 (s, 2H), 4.40 (s, 2H), 3.70-3.59 (m, 2H), 3.42-3.38 (m, 2H), 3.13 (s, 3H), 2.20 (m, 4H), 1.49 (m, 4H).

The comparison of ¹H-NMR of cocrystal Form III and the Lapatinib·HCl salt indicates that there are not significant shifts in the different proton signals belonging to Lapatinib. Therefore, a salt of Lapatinib·HCl with adipic acid does not seem to be formed in DMSO.

- DSC: DSC analysis was recorded using a Mettler DSC822e instrument. A sample of 1.2150 mg was weighed into a 40 µL aluminum crucible with a pinhole lid and was heated, under nitrogen (50 mL/min), at 10°C/min from 30 to 300°C.

Form III is characterized by an endothermic sharp peak corresponding to the melting point with an onset at 188.87°C (fusion enthalpy -81.35 J/g), measured by DSC analysis (10 °C/min).

- TGA: Thermogravimetric analysis was recorded in a thermogravimetric analyzer Mettler TGA/SDTA851^{e}. A sample of 7.4451 mg was weighed into a 70 µL alumina crucible with a pinhole lid and was heated at 10 °C/min from 30 to 300°C, under nitrogen (50 mL/min).

The TG analysis of Form III only shows a negligible weight loss (0.16 %) before the melting point.

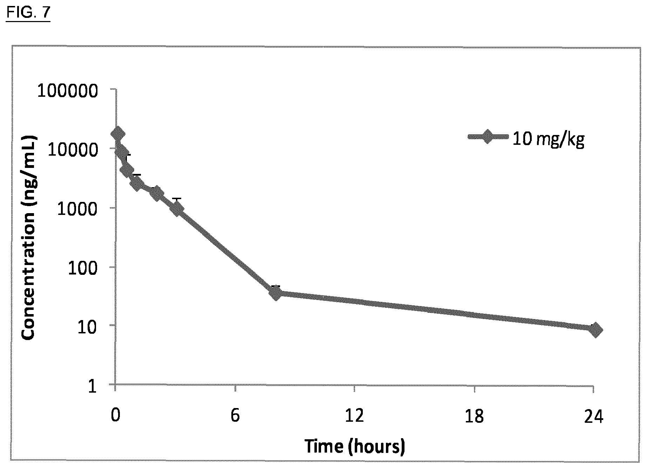

- XRPD: XRPD analysis was performed using a PANalytical X'Pert diffractometer with Cu Kα radiation in Bragg-Brentano geometry. The system is equipped with a monodimensional, real time multiple strip detector. The diffractogram was recorded from 3° to 40° (2θ) at a scan rate of 17.6° per minute (see Figure 7). Peaks with relative intensity greater than or equal to 1 % are indicated in the Table below:

| Pos. [°2Th] | d-spacing [Å] | Rel. Int. [%] |
|---|---|---|
| 6,15 | 14,37 | 18 |
| 7,79 | 11,35 | 24 |
| 9,97 | 8,88 | 21 |
| 11,80 | 7,50 | 7 |
| 12,46 | 7,10 | 46 |
| 12,91 | 6,86 | 33 |
| 14,32 | 6,18 | 15 |
| 14,78 | 6,00 | 30 |
| 15,77 | 5,62 | 60 |
| 17,25 | 5,14 | 46 |
| 19,00 | 4,67 | 100 |
| 19,44 | 4,57 | 39 |
| 20,00 | 4,44 | 40 |
| 20,43 | 4,35 | 33 |
| 21,10 | 4,21 | 62 |
| 21,70 | 4,10 | 77 |
| 22,07 | 4,03 | 39 |
| 22,78 | 3,90 | 17 |
| 23,76 | 3,74 | 27 |
| 25,30 | 3,52 | 23 |
| 26,07 | 3,42 | 22 |
| 26,92 | 3,31 | 43 |
| 28,71 | 3,11 | 23 |
| 31,02 | 2,88 | 12 |
| 31,88 | 2,81 | 12 |

The co-crystal of Lapatinib being Lapatinib monohydrochloride : adipic acid (1:1) of formula (Form III) has a X-Ray Powder Diffractogram (XRPD) with characteristic peaks expressed in 2 Theta value of: 6,15 (m), 7,79 (m), 9,97 (m), 12,46 (s), 12,91 (m), 14,32 (m), 14,78 (m), 15,77 (s), 17,25 (s), 19,00 (vs), 19,44 (m), 20,00 (m), 20,43 (m), 21,10 (s), 21,70 (s), 22,07 (m), 22,78 (m), 23,76 (m), 25,30 (m), 26,07 (m), 26,92 (s), 28,71 (m), 31,02 (m), 31,88 (m), (+/-) 0,10; wherein (vs) = very strong intensity, (s) = strong intensity, (m) = medium intensity. In particular, the co-crystal having formula (Form III) has characteristic peaks expressed in 2 Theta value of: 15,77, 17,25, 19,00, 21,10, 21,70, 26,92 (+/-) 0,10.

### Example 7 - Preparation of Lapatinib monotosylate of formula (I, 1 TsOH).

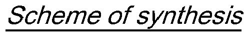

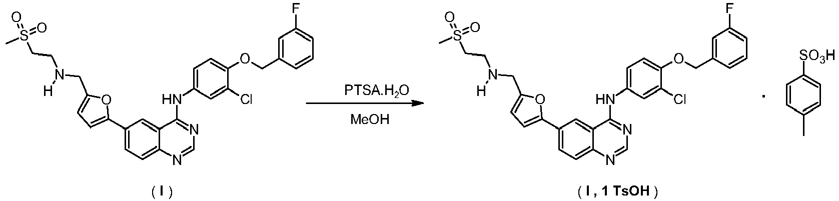

Lapatinib free base (8.00 g, 13.77 mmol) was suspended in MeOH (320 mL, 40 volumes). To the brown/yellow suspension, PTSA·H2O (2.68 g, 14.09 mmol, 1.02 eq) was added and the resulting bright yellow mixture was stirred at r.t. overnight. The solid was filtered, washed with MeOH (40 mL, 5 volumes) and dried under vacuum at r.t., affording Lapatinib monotosylate (9.73 g, 12.92 mmol, 94% yield) as a yellow solid.

### Example 8 - Preparation of Lapatinib monohydrochloride of formula (I, 1 HCl).

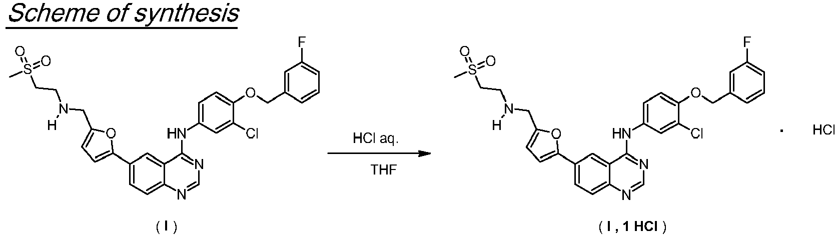

Lapatinib free base (13.00 g, 22.37 mmol) was suspended in THF (260 mL, 20 volumes). To the brown/yellow suspension, HCl 1 N (22.4 mL 22.40 mmol, 1.0 eq) was added and the resulting bright yellow mixture was stirred at r.t. overnight. The solid was filtered, washed with THF (26 mL, 2 volumes) and dried under vacuum at r.t., affording Lapatinib monohydrochloride (13.24 g, 21.44 mmol, 96% yield) as a yellow solid.

### Example 9 - Preparation of co-crystal Lapatinib monotosylate : caproic acid (1:2) of formula (Form I):

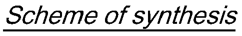

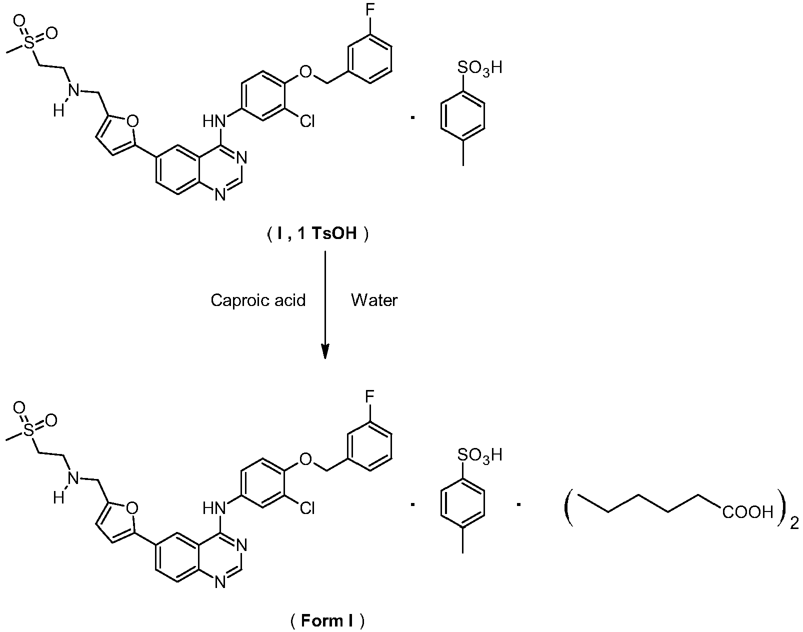

Lapatinib monotosylate (4.01 g, 5.32 mmol) was suspended in H₂O (80 mL, 20 volumes). Caproic acid (1.40 mL, 0.927 g/mL, 11.17 mmol, 2.1 eq) was added and the resulting suspension was stirred at r.t. overnight. A sample was filtered and analyzed by XRPD. Cocrystal Form I was obtained with residual Lapatinib monotosylate. Additional caproic acid (0.27 mL, 0.927 mg/mL, 2.15 mmol, 0.4 eq.) was added and the slurrying was stirred for a further 4 h. The solid was filtered, washed with water (10 mL, 2.5 volumes) and dried under vacuum at r.t., affording the desired cocrystal (4.84 g, 92% yield) as an off-white solid.

### Example 10 - Preparation of co-crystal Lapatinib monotosylate : caprylic acid (1:2) of formula (Form II):

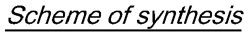

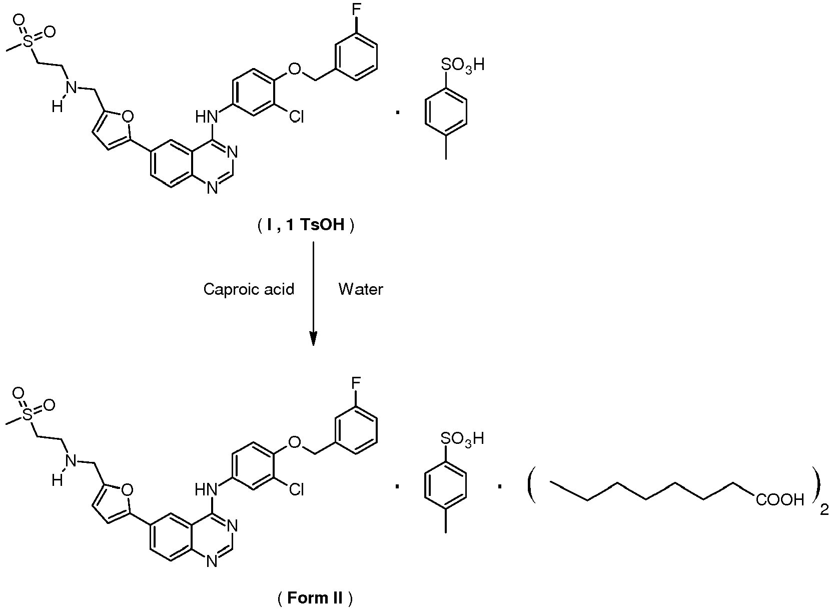

Lapatinib monotosylate (4.00 g, 5.31 mmol) and caprylic acid (1.85 mL, 0.910 g/mL, 11.67 mmol, 2.2 eq.) were suspended in H₂O:EtOH 5:1 (80 mL, 20 volumes). The resulting suspension was stirred at r.t. overnight. A sample was filtered and analyzed by XRPD. Cocrystal Form 2 was obtained with residual Lapatinib monotosylate. Additional caprylic acid (0.084 mL, 0.910 mg/mL, 0.53 mmol, 0.1 eq.) was added, and additional solvent (12 mL, 3 V, H₂O:EtOH 5:1), and the slurrying was stirred over the weekend. The solid was filtered, washed with water (3 x 12 mL, 3 x 3 volumes) and dried under vacuum at r.t., affording the desired cocrystal (5.08 g, 92% yield) as an off-white solid.

### Example 11 - Preparation of co-crystal Lapatinib monohydrochloride : adipic acid (1:1) of formula (Form III)), which is not part of the present invention:

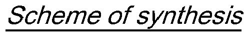

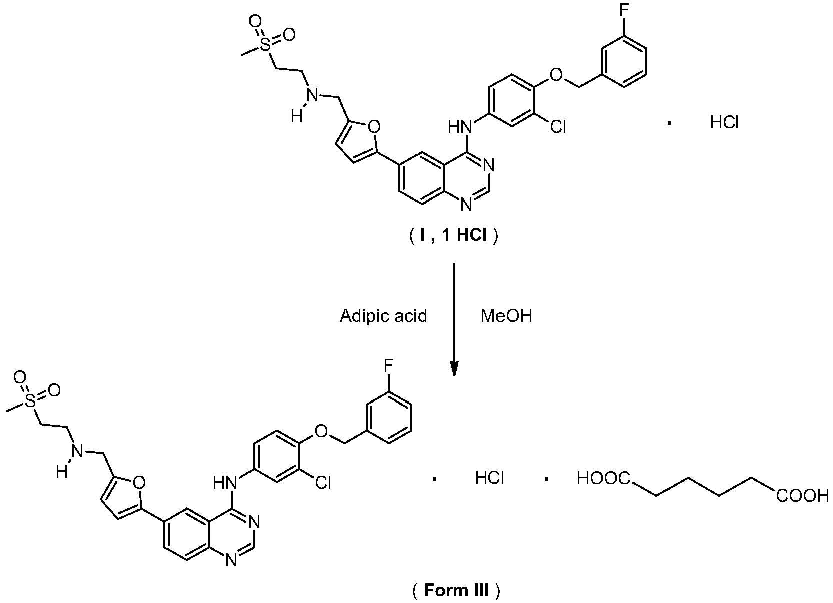

Lapatinib monohydrochloride (4.05 g, 6.56 mmol,) and adipic acid (2.02 g, 13.82 mmol, 2.1 eq) were suspended in MeOH (80 mL, 20 volumes). The resulting suspension was stirred overnight at r.t. The solid was filtered, washed with MeOH (3 x 12 mL, 3 x 3 volumes) and dried under vacuum, affording form III (4.26 g, 85% yield) as a pale yellow solid.

### Example 12 - Preparation of Lapatinib monotosylate of formula (I, 1 TsOH).

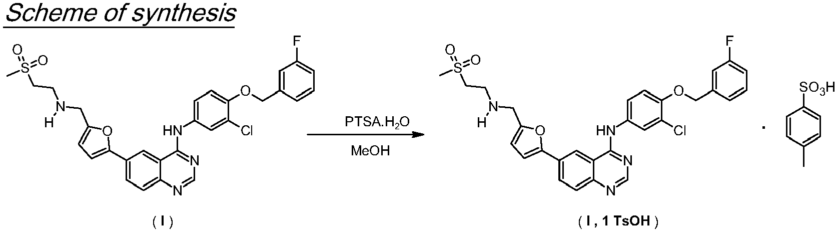

Lapatinib free base (17.26 g, 29.70 mmol) was suspended in MeOH (690 mL, 40 volumes). To the brown/yellow suspension, PTSA·H2O (5.76 g, 30.28 mmol, 1.02 eq) was added and the resulting bright yellow mixture was stirred at RT overnight. The solid was filtered, washed with MeOH (86 mL, 5 volumes) and dried under vacuum at RT, affording Lapatinib monotosylate (21.02 g, 27.91 mmol, 94% yield) as a yellow solid.

### Example 13 - Preparation of Lapatinib monohydrochloride of formula (I, 1 HCl).

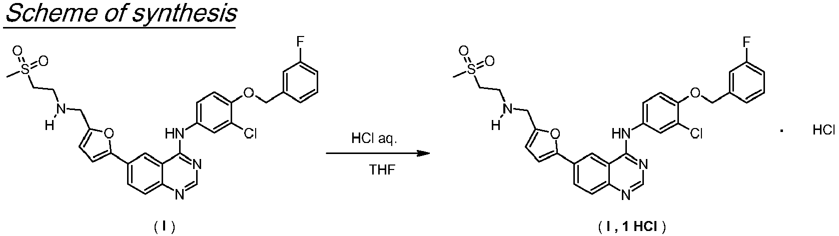

Lapatinib free base (13.00 g, 22.37 mmol) was suspended in THF (260 mL, 20 volumes). To the brown/yellow suspension, HCl 1 N (22.4 mL 22.40 mmol, 1.0 eq) was added and the resulting bright yellow mixture was stirred at RT overnight. The solid was filtered, washed with THF (26 mL, 2 volumes) and dried under vacuum at RT, affording Lapatinib monohydrochloride (13.24 g, 21.44 mmol, 96% yield) as a yellow solid.

### Example 14 - Preparation of co-crystal Lapatinib monotosylate : caproic acid (1:2) of formula (Form I):

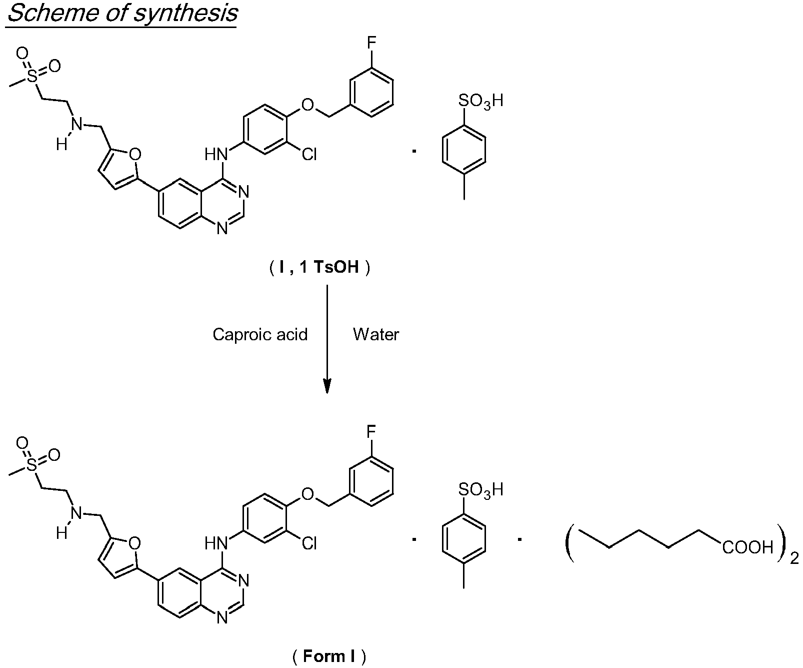

Lapatinib monotosylate (13.50 g, 17.92 mmol) was suspended in H₂O (270 mL, 20 volumes). Caproic acid (5.6 mL, 0.927 g/mL, 44.69 mmol, 2.5 eq) was added and the resulting suspension was stirred at RT overnight. A sample was filtered and analyzed by XRPD. Cocrystal Form I was obtained with residual Lapatinib monotosylate. Additional caproic acid (0.45 mL, 0.927 mg/mL, 3.59 mmol, 0.2 eq.) and more solvent (27 mL, 2 volumes) were added and the slurrying was stirred for 4 h (at this point the stirring was not good). The solid was filtered, washed with water (2 x 34 mL, 2 x 2.5 volumes) and dried under vacuum at RT, affording the desired cocrystal (16.83 g, 95% yield) as an off-white solid.

### Example 15 - Preparation of co-crystal Lapatinib monotosylate : caprylic acid (1:2) of formula (Form II):

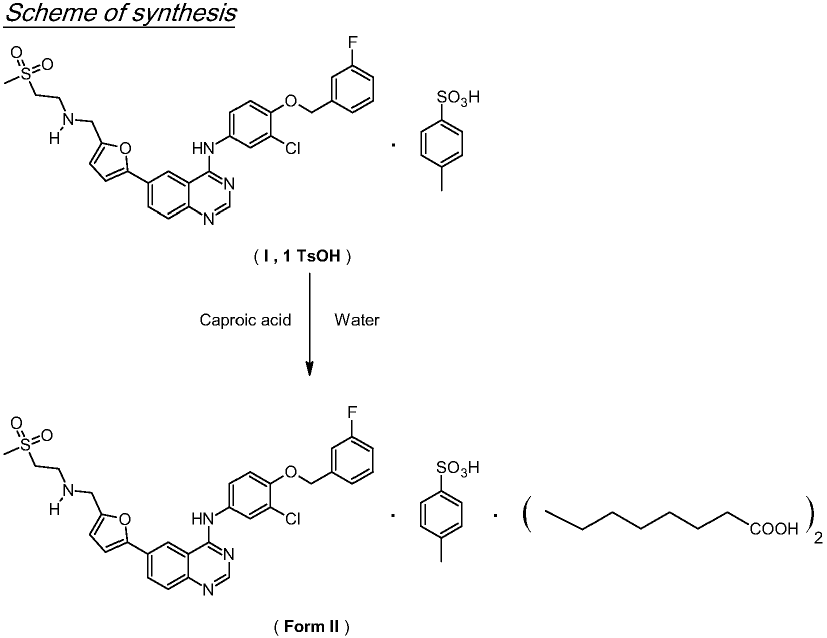

Lapatinib monotosylate (13.50 g, 17.92 mmol) and caprylic acid (6.5 mL, 0.910 g/mL, 41.02 mmol, 2.3 eq.) were suspended in H₂O:EtOH 5:1 (270 mL, 20 volumes). The resulting suspension was seeded with Form II and stirred at RT overnight. A sample was filtered and analyzed by XRPD. Co-crystal Form II was obtained with residual Lapatinib monotosylate. The slurrying was continued for 3.5 h (22 h in total) and complete transformation was checked by XRPD. The solid was filtered, washed with H₂O:EtOH 5:1 (3 x 40.5 mL, 3 x 3 volumes) and dried under vacuum at RT, affording the desired co-crystal (17.63 g, 94% yield) as an off-white solid.

Different samples of this solid were analyzed by XRPD and residual Lapatinib monotosylate was detected in some of them. As the solid was not homogeneous and residual starting product was detected the solid was reprocessed.

### Example 16 - Preparation of co-crystal Lapatinib monotosylate : caprylic acid (1:2) of formula (Form II)), which is not part of the present invention - reprocessing:

Lapatinib monotosylate:caprylic acid 1:2 (17.63, 16.92 mmol) and caprylic acid (2.7 mL, 0.910 g/mL, 17.04 mmol, 1 eq.) were suspended in H₂O:EtOH 5:1 (350 mL, 20 volumes). The resulting suspension was stirred at RT overnight. A sample was filtered and analyzed by XRPD confirming the transformation of residual Lapatinib monotosylate. The solid was filtered, washed with H₂O:EtOH 5:1 (8 x 70 mL, 8 x 4 volumes) and dried under vacuum at RT, affording the desired cocrystal as an off-white solid. The solid was analyzed by 1 H-NMR and an excess of almost 1 equivalent of caprylic acid was detected. The solid was resuspended twice in H₂O:EtOH 5:1 (350 mL, 20 volumes) until the excess of caprylic acid was completely removed from the solid. The desired cocrystal (16.09 g, 86% overall yield) was obtained as an off-white solid.

### Example 17 - Preparation of co-crystal Lapatinib monohydrochloride : adipic acid (1:1) of formula (Form III)), which is not part of the present invention:

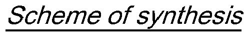

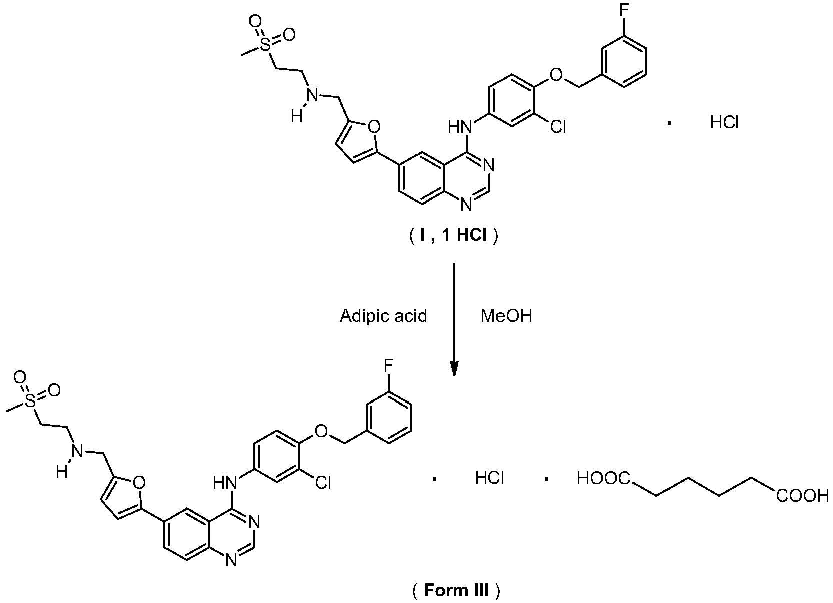

Lapatinib monohydrochloride (4.93 g, 7.98 mmol) and adipic acid (2.31 g, 15.81 mmol, 2 eq) were suspended in MeOH (98 mL, 20 volumes). The resulting suspension was stirred overnight at RT. The solid was filtered, washed with IPA (3 x 15 mL, 3 x 3 volumes) and dried under vacuum, affording Form III (5.33 g, 87% yield) as a pale yellow solid. This experiment furnished Form III as a pure form without excess of adipic acid (confirmed by XRPD and ¹H-NMR).

### Example 18 - Stability Study - Stability in water suspension.

Stability of Form I, Form II and Form III in a water suspension was studied.

Co-crystal Lapatinib monotosylate : caproic acid (1:2) is Form I,
Co-crystal Lapatinib monotosylate : caprylic acid (1:2) is Form II,
Co-crystal Lapatinib monohydrochloride : adipic acid (1:1) is Form III;

The three suspensions were stirred at room temperature in 40 volumes of water and the transformation of the product was followed by XRPD at 1 hour, 4 hours and 24 hours. Form II, i.e. co-crystal Lapatinib monotosylate : caprylic acid (1:2), is the most stable co-crystal, recovering pure Form II after 24 hours, while Form III and Form I are less stable in water; a little of Lapatinib Tosylate or Lapatinib hydrochloride was detected after 1 hour.

### Example 19 - Stability study at controlled humidity and temperature conditions.

Two different humidity/temperature conditions were selected for this study:
a. High humidity (94 RH%) at room temperature; and
b. Accelerated conditions of ICH guideline (40°C - 75 RH%).

### a. 94% RH at room temperature

No crystalline changes were detected by XRPD of any of the three forms after 41 days at 94% RH at room temperature. These co-crystals are quite stable at room temperature and high relative humidity. After 115 days under 94% RH at room temperature, the XPRD analysis confirms the complete stability of all three co-crystals (form I, II and III) under said conditions.

No crystalline changes were detected by XRPD of any of the three forms I, II and III after 115 days at 94% RH at room temperature. These co-crystals are stable at room temperature and high relative humidity.

### b. 74% RH at 40°C

No crystalline changes were detected by XRPD of any of the three forms after 11 days at 74% RH at 40°C.

After 23 days Lapatinib monotosylate is observed in the co-crystals of formula (Form II) (only traces) and co-crystals of formula (Form I) (a little amount).

Very small amounts of Lapatinib monotosylate Form M1 (solid form as disclosed in WO2010027848A2) were detected by XRPD in Form I and Form II respectively after 23 and 60 days of exposure at 40 °C with 74% RH. Form M1 increased very slowly with time.

However, no crystalline changes were detected by XRPD to Form III after 3 months at 74% RH at 40°C. Therefore the Form III is the only one which remained stable after 97 days at 74% RH at 40°C.

The conclusions of the stability studies at controlled humidity and temperature conditions are: the co-crystals of Lapatinib of the present inventions are stable, thus suitable for pharmaceutical and medical uses. Furthermore, the ranking of stability at 40°C with 74% RH is the following: co-crystal of formula (Form III) > co-crystal of formula (Form II) > co-crystal of formula (Form I), thus the co-crystal of formula (Form III) is the one more stable.

### Example 20 - Solubility studies

Stability tests of Form I, Form II and Form III in a water suspension indicated that these co-crystals dissociate in water, especially Form I. Therefore the solubility study was performed reducing the time slurrying in water to 1 h with the three co-crystals, Forms I, II and III and Lapatinib ditosylate. Analysis by HPLC of these products afforded good purities (ca. 99-100% a/a).

The Lapatinib salts are very insoluble in water and so it is not possible to directly analyze the mother liquors of the slurrying in order to determine the solubility (Lapatinib concentration is below the detection limit of HPLC method). Therefore these mother liquors had to be concentrated leading to possible error (for example, loss of products over the wall of the flask).

To overcome this problem, the solubility test has been carried out in DMSO and precipitating the salt/cocrystal with water. Lapatinib in the filtered solution should be sufficiently soluble to be directly analyzed by HPLC. However, as cocrystals Forms I, II and III were not obtained by precipitation in DMSO/water these solubility tests were discarded.

During the solubility test (slurrying + filtration + evaporation to dryness) some problems of reproducibility were found, caused by:

Problems of filtration. The lack of reproducibility of some solubility tests seems to be due to filtration problems due to the presence of very small particles. These particles (not visible with the naked eyed) seem to be progressively stopped by successive filtration (it was also observed that these small yellowish crystals pass through the HPLC filter). In order to check these hypotheses, the yellowish clear solution coming from the first filtration was centrifuged at 14000 rpm affording a yellowish solid in the bottom of the tube and a clearer solution. Therefore all the solutions were repeatedly filtered until centrifugation of an aliquot confirmed the elimination of these particles.

Problem of degradation. Degradation of Lapatinib during the evaporation process at 45 °C under vacuum was observed by HPLC. In some cases only degradation products were recovered (solubility tests Forms I and II). Therefore, the HPLC area cannot be used to determine the solubility of Lapatinib. The same solubility tests with Form II lowering the evaporation temperature and reducing the time, allowed recovery of Lapatinib as major product. As the weight seems to be similar in both cases, it could be considered in a primary approximation as the weight of pure Lapatinib salt + coformers.

Based on the recovered weights coming from the solubility tests (considering that the degradations did not lead to weight variation and using a correction factor depending on the molecular mass of the salt/cocrystals to obtain the corresponding mass of Lapatinib free base) it can be primarily established that:
- Form II and Lapatinib ditosylate have a similar solubility; and
- Form I and Form III have a higher solubility than Lapatinib ditosylate.
- The most soluble product is co-crystal Form III (approximately four times more soluble than the ditosylate salt).

To improve the accuracy of these results the evaporation process could be repeated faster for the solubility tests of Forms I and III.

Furthermore, the ranking of solubility is the following: co-crystal of formula (Form III) > co-crystal of formula (Form I) > co-crystal of formula (Form II) having about the same solubility of Lapatinib ditosylate.

Co-crystal Form III (Lapatinib·HCl·Adipic acid (1:1)) is the form offering highest stability and solubility.

Co-crystals of Lapatinib tosylate indicated that Form I is more soluble than Form II. This trend is similar with the solubility of the co-former Caproic acid which is much more soluble than Caprylic acid in water. However, the lower solubility of Form II seems to increase the stability of the crystalline form in humidity conditions (40 °C, 74% RH and water slurrying).

### Example 21 - Pharmacokinetic and Bioavailability studies

### Definitions and Abbreviations - Table 1

**Table 1:**

| | |
|---|---|
| AUCinf | Area under the concentration-time curve from 0 to infinity |
| AUCt | Area under the concentration-time curve from 0 to the last measurable concentration |
| AUMCinf | Area under the first moment concentration-time curve from 0 to infinity |
| AUMCt | Area under the first moment concentration-time curve from 0 to the last measurable concentration |
| C0 | Extrapolated concentration at time zero (iv) |
| CL | Plasma clearance (iv) |
| CL/F | Plasma clearance normalized by the fraction of dose absorbed (po) |
| Cmax | Maximum concentration (po) |
| Ct | Plasma concentration at time t |
| CV | Coefficient of Variation |
| FDA | Food and Drug Administration |
| iv | Intravenous |
| λz | Terminal phase rate constant (iv) |
| λz app | Apparent first order elimination rate constant associated to the terminal phase (po) |
| LLOQ | Lower Limit of Quantification |
| MRTinf | Mean residence time from zero to infinity |
| MRTt | Mean residence time from zero to the last quantifiable experimental time |
| OECD | Organization for Economic Co-operation and Development |
| po | Oral administration |
| QC | Quality Control |
| r | Correlation coefficient |
| RE | Relative Error |
| SOP | Standard Operating Procedure |
| t1/2 | Terminal elimination half-life (iv) |
| t1/2 app | Apparent terminal elimination half-life (po) |
| ULOQ | Upper Limit of Quantification |
| UPLC®- | Ultra-Performance Liquid Chromatography with Tandem |
| MS/MS | Mass Spectrometry |
| Vss | Volume of distribution at steady-state (iv) |
| Vz | Volume of distribution at the terminal disposition phase (iv) |
| Vz/F | Volume of distribution at the terminal disposition phase normalized by the fraction of dose absorbed (po) |
| WFI | Water for injection |

### Body Weights, Doses and Blood Sampling Times

For the preparation of the intravenous solution and the oral suspensions, the correction factor and the purity were applied for all compounds. Blood samples were collected from all animals at all scheduled time points.

### Plasma Levels

The study samples were analyzed according to the following UPLC^{®}-MS/MS method:

| | |
|---|---|
| HPLC Column: | XTerra® RP18 5µm particle size 50 x 4.6 mm (Waters) |
| Column temperature: | 40 °C |
| Injector temperature: | 4 °C |
| Run-time: | 4 minutes |
| Injection volume: | 20 µL |
| Column flow rate: | 0.8 mL/min |
| Purge solvent/Weak solvent: | 0.1 % formic acid in H2O |
| Autosampler wash solution / | Methanol:H₂O:ACN:Isopropanol (1:1:1:1) and Formic Acid 1% |

**Strong Solvent:**

| | Solvents | A: | Acetonitrile | | | |
|---|---|---|---|---|---|---|
| | | B: | 0.1% formic acid in H2O | | | |
| Mobile phase: | Gradient elution time (min) | | A% | B% | Flow (mL/min) | Curve* |
| | 0.00 | | 20 | 80 | 0.8 | - |
| | 0.50 | | 20 | 80 | 0.8 | 6 |
| | 1.50 | | 95 | 5 | 0.8 | 6 |
| | 2.00 | | 95 | 5 | 0.8 | 6 |
| | 4.00 | | 20 | 80 | 0.8 | 1 |
| Retention time: | Lapatinib: ∼ 1.5 min | | | | | |

| | | | | | | |
|---|---|---|---|---|---|---|
| Wherein * 1: Isocratic mode 6: Linear mode | | | | | | |

### Intravenous Administration

The individual and mean ± SD plasma Lapatinib levels obtained after the intravenous administration of Lapatinib ditosylate monohydrate in DMSO:PEG:WFI (10:40:50) at 10 mg/kg to male rats are shown in Fig. 7.

Following an intravenous bolus administration at 10 mg/kg, mean plasma levels declined as a function of time showing two disposition phases (from the first sampling time up to 3 hours postdose, and from 8 to 24 hours postadministration). However, the fact that only two time points corresponding to this second phase (terminal elimination phase) were obtained did not allow the calculations of the pharmacokinetic parameters derived from this terminal phase (AUC_{inf}, t_{1/2}, MRT, V_{z}, Vₛₛ and CL).

### Oral Administrations

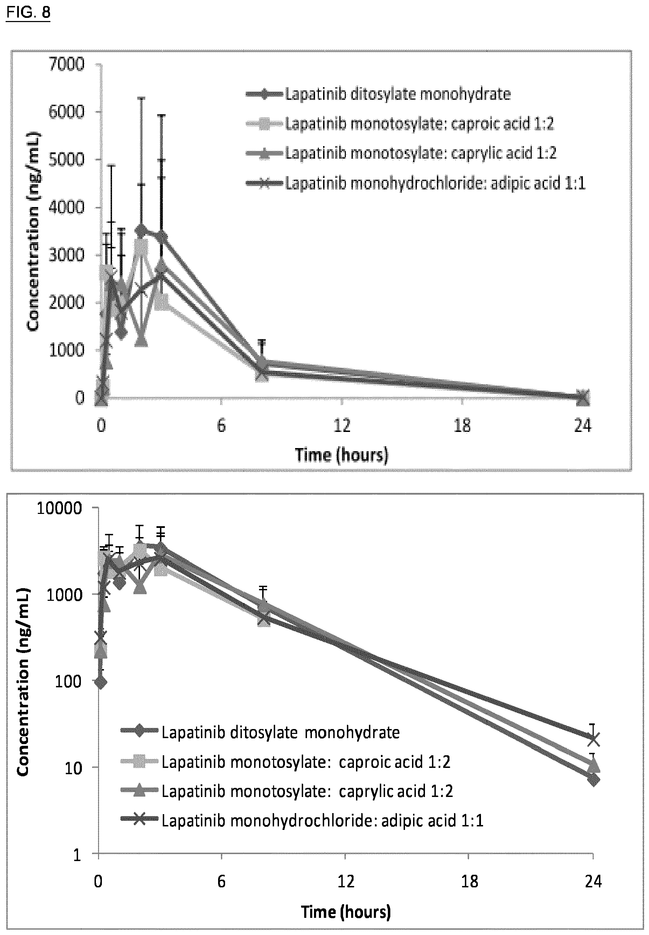

The individual and mean plasma Lapatinib concentrations obtained after its oral administration as a ditosylate monohydrate (comparative form) or as three different solid crystal formulations in hydroxypropylmethylcellulose : Tween 80 (0.5%:0.1%) in WFI at 30 mg/kg to male rats are represented in linear and semi-logarithmic plots in Fig. 8.

The plasma profiles obtained for all the compounds were similar. There was a rapid absorption phase (peak plasma levels reached at 2 or 3 hours postdose) followed by fast decay.

### Exposure and Pharmacokinetic Parameters:

### - Intravenous Administration

Pharmacokinetic parameters obtained for Lapatinib after its intravenous administration show that the extrapolated concentration at time 0 (Co) and for AUCₜ were 20222 ng/mL and 13661 ng·h/mL, respectively. Moreover, MRTₜ was 1.54 hours.

### - Oral Administration

Exposure and pharmacokinetic parameters obtained for Lapatinib after its oral administration as a ditosylate monohydrate (comparative form) or as three different solid crystal formulations, as a summary of mean ± SD values is shown in the following Table 2:

**Table 2**

| **Lapatinib** | | | | |
|---|---|---|---|---|
| **Parameter** (Units) | *Lapatinib ditosylate monohydrate* | *Lapatinib monotosylate: caproic acid 1:2* | *Lapatinib monotosylate: caprylic acid 1:2* | *Lapatinib monohydrochl oride: adipic acid 1:1* |
| **Cₘₐₓ** (ng/mL) | 3521 | 3171 | 2814 | 2567 |
| **tₘₐₓ** (h)^{*} | 2 | 2 | 3 | 3 |
| **AUCₜ** (ng*h/mL) | 23561 | 13287 | 20778 | 18433 |
| **Cₘₐₓ/AUCₜ** (h⁻¹) | 0.149 | 0.239 | 0.135 | 0.139 |
| **t**_{1/2app} (h) | 2.39 | 2.37 | 2.62 | 3.13 |
| **MRTₜ** (h) | 4.28 | 2.77 | 4.67 | 4.29 |
| CL/F | 1272 | 1995 | 1441 | 1620 |
| **V_{z}/F** | 4384 | 6831 | 5444 | 7314 |
| **F** (%) | 57.49 | 32.42 | 50.70 | 44.98 |

The highest peak plasma Lapatinib levels were reached by the Lapatinib ditosylate monohydrate and the co-crystal Lapatinib monotosylate: caproic acid 1:2.

However, the highest AUC values were obtained for the Lapatinib ditosylate monohydrate (comparative form) and the co-crystal Lapatinib monotosylate: caprylic acid 1:2. Consequently, the comparison of AUCₜ values and the intravenous administration resulted in a higher absolute bioavailability for these two solid forms than for the other co-crystal formulations. Furthermore, Lapatinib ditosyalte monohydrate and the co-crystal Lapatinib monotosylate: caprylic acid 1:2, considering the confidence interval, have substantially the same bioavailability.

Values of the apparent absorption rate (Cₘₐₓ/AUCₜ) were similar for all the compounds. Nevertheless the co-crystal Lapatinib monotosylate: caproic acid 1:2 showed a higher value (even in comparison with Lapatinib ditosylate monohydrate), which indicated a faster absorption of Lapatinib when Lapatinib is in such a solid form. Therefore, co-crystal Lapatinib monotosylate: caproic acid 1:2 is suitable for immediate or fast-release composition of Lapatinib.

No major differences were observed in the apparent elimination half-lives obtained for Lapatinib after the administration of all the compounds. Lapatinib monotosylate: caprylic acid 1:2 and Lapatinib monohydrochloride: adipic acid 1:1 showed the longest half-lives (2.62 and 3.13 h, respectively).

Mean residence times (MRTt) were also similar after the administration of all the compounds (ranging from 4.28 to 4.67 h), except for the monotosylate: caproic acid 1:2, which showed a lower value (2.77 h).

Clearance and volume of distribution are primary parameters obtained after the intravenous administration. Since they could not be correctly obtained, the clearance and volume of distribution at the terminal disposition phase normalized by the fraction of dose absorbed (CL/F and Vz/F, respectively) were calculated after the oral administration of the different Lapatinib solid forms.

CL/F values obtained for ditosylate monohydrate (comparative form) and the monotosylate: caprylic acid 1:2 were lower than those obtained for the other crystal formulations, which indicated a slower elimination of the compound from these formulations.

The highest value of V_{z}/F was obtained for Lapatinib monohydrochloride: adipic acid 1:1. However, this parameter is only representative of the distribution of the compound at the end of the distribution phase (when elimination phase is dominant).

From all the tested solid forms, Lapatinib ditosylate monohydrate (marketed form) and the Lapatinib monotosylate: caprylic acid 1:2 seemed to be the most favorable in terms of absolute bioavailability. The elimination of Lapatinib from these two formulations seemed slower than from the rest of co-crystals.

Finally, slightly longer apparent elimination half-life and mean residence time were obtained for the monotosylate: caprylic acid 1:2 compared to the ditosylate monohydrate (marketed form).

The plasma Lapatinib concentration obtained after 5 min. from the oral administration of one of the following Lapatinib solid forms in hydroxypropylcellulose : Tween 80 (0.5% : 0.1 %) in water for injection at 30 mg/kg to male rats is summarized in Table 3:

**Table 3**

| Compound | Concentration (ng/mL) |
|---|---|
| Lapatinib ditosylate monohydrate | 98 ± 34 |
| Co-crystal Lapatinib monotosylate : caproic acid (1:2) | 228 ± 95 |
| Co-crystal Lapatinib monotosylate : caprylic acid (1:2) | 324 ± 23 |
| Co-crystal Lapatinib monohydrochloride : adipic acid (1:1) | 320 ± 56 |

Analyzing the data of Table 3, it is clear that all the three co-crystals of Lapatinib provides much higher plasmatic concentration after 5 minutes by the oral administration in comparison with the reference Lapatinib ditosylate monohydrate.

Said effect at very short time after the administration is perhaps due to the higher permeability shown by co-crystal of the present invention, likely due to the "fat vehicle" of the fatty acid co-former.

Therefore, the co-crystal of Lapatinib monoacid salts of the present invention can be used for immediate release oral pharmaceutical form or oral immediate release dosage forms.

### Example 22 - Pharmacokinetic and Bioavailability studies

Evaluation of the Pharmacokinetic (PK) characteristics and the bioavailability of Lapatinib when administered orally as a ditosylate monohydrate salt or as three different solid co-crystals of Lapatinib.

A model-based approach was applied to characterize the PK profiles and the bioavailability of Lapatinib when administered intravenously and orally as a ditosylate monohydrate or as three different solid crystal formulations in rats.

The PK model was used to compare the rate and extent of absorption of Lapatinib when administered orally as a ditosylate monohydrate or as three different solid crystal formulations, i.e. the three co-crystals of Lapatinib of the present invention of formula (Form I), (Form II) and (Form (III)).

The mean plasma concentration observed at the different sampling times was used in the analyses.

### Intravenous (IV) administration

The structural model for Lapatinib reference products was described by a three-compartment model characterized by a central compartment and a slow and fast peripheral distribution compartments.

A two compartment model was also applied but the result of the fitting procedures indicates that the 3-compartment model performed statistically better. Therefore the 3-compartment model was finally retained.

### Oral administration

The analysis of the oral absorption PK profile of Lapatinib indicated the presence of a dual peak.

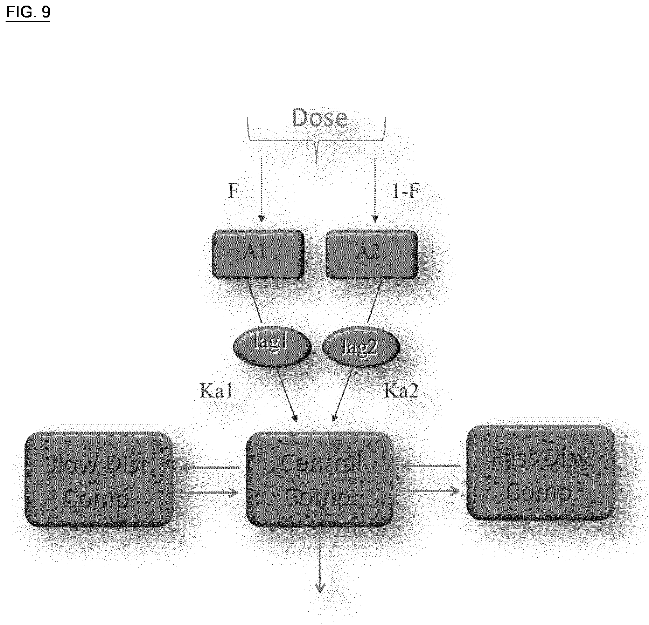

Therefore, the absorption process was described by two parallel inputs processes. The first input characterized a fast-release component (with a ka1 rate of absorption) while the second input characterized a delayed release of the drug (with a ka2 rate of absorption). The fraction of the oral dose associated with the first absorption component was estimated by the F parameter, while the remaining fraction of the drug (1-F) was assumed to be released in the second absorption process. The F fraction of the dose was immediately release (lag1) while the release of the remaining (1-F) fraction occurred with an estimated lag time (lag2). The structure of the oral PK model is presented in Figure 9.

The disposition and elimination PK parameters of Lapatinib (k12, k21, k13, k31, and kel) for the different oral formulations were fixed to the ones estimated in the IV model due to the limited PK samples available for a proper characterization of these parameters after oral administration.

### Software

Data preparation and graphical display presentation was performed using R (version 3.0.2). The R scripts used for data preparation and final analyses were achieved. The PK analysis was conducted using the NONMEM^{®} software, Version 7.3 (ICON Development Solutions).

### Results

Table 4 shows the treatment groups:

| Group | Compound |
|---|---|
| 1 | Lapatinib ditosylate monohydrate |
| 2 | Lapatinib ditosylate monohydrate |
| 3 | co-crystal Lapatinib monotosylate : caproic acid (1:2) of formula (I), |
| 4 | co-crystal Lapatinib monotosylate : caprylic acid (1:2) of formula (Form II), |
| 5 | co-crystal Lapatinib monohydrochloride : adipic acid (1:1) of formula (Form III); |

Table 5 shows the estimated parameters in the IV administration and Oral models with different treatment groups.

**Table 5**

| **Group 1** | | | | **Group 2** | **Group 3** | **Group 4** | **Group 5** |
|---|---|---|---|---|---|---|---|
| kel | 1.6 | | F1 | 0.295 | 0.426 | 0.505 | 0.885 |
| k12 | 0.616 | | lag1 | 0 | 0 | 0 | 0 |
| k21 | 0.00202 | | lag2 | 1.57 | 1 | 2.14 | 2.2 |
| v | 0.358 | | ka1 | 1.09 | 1.15 | 0.587 | 0.358 |
| k13 | 3.14 | | ka2 | 0.509 | 0.423 | 0.426 | 4.4 |
| k31 | 1.47 | | v | 0.741 | 0.916 | 0.811 | 0.894 |

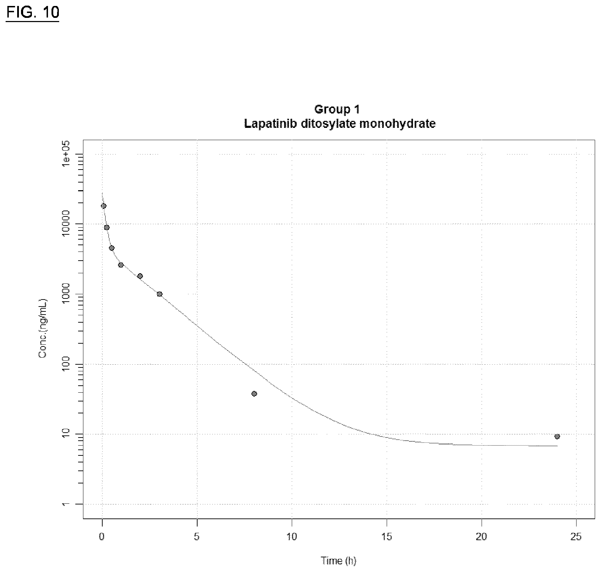

Figure 10 show the scatter plot of the observed data with the model prediction after IV administration.

Figure 11 show the scatter plot of the observed data with the model prediction after oral administration by treatment group.

In table 6 are summarized the relevant pharmacokinetic parameters derived from the pharmacokinetic modelling results by treatment group.

| **Group** | **Cmax (ng/mL)** | **AUC0t (ng*h/mL)** | **AUC0INF (ng*h/mL)** | **Tmax (h)** | **MRTINF (h)** | **T12 (h)** | **Absolute Bioavail. (%)** |
|---|---|---|---|---|---|---|---|
| 1 | | 12300 | 14100 | | 35.9 | 176 | |
| 2 | 3511.6 | 18400 | 18400 | 2 | 4.31 | 2.28 | 43.5 |
| 3 | 3018.6 | 14900 | 15000 | 1.6 | 3.85 | 2.38 | 35.5 |
| 4 | 2813.5 | 16600 | 16700 | 3 | 4.67 | 2.32 | 39.5 |
| 5 | 2237.4 | 13500 | 13600 | 1.6 | 4.35 | 2.59 | 32.2 |

### Conclusions

The PK models describe reasonably well the observations. However, the characterization of the disposition and elimination processes of Lapatinib after IV and after the different treatment administrations has to be cautiously considered. This is due to the limitations in the experimental design of the study conducted in rats that did not properly covered the time course of the Lapatinib in the interval 8 to 24 hours post-dose. The estimated parameters indicated that the pharmacokinetic properties of the different treatments are very similar with a more pronounced similarity between group 4, i.e. the co-crystal Lapatinib monotosylate : caprylic acid (1:2) of formula (Form II) and group 2, i.e. Lapatinib ditosylate monohydrate.

## Claims

1. Co-crystal of Lapatinib selected in the group consisting of:
(a) Lapatinib monotosylate : caproic acid (1:2) of formula (Form I): 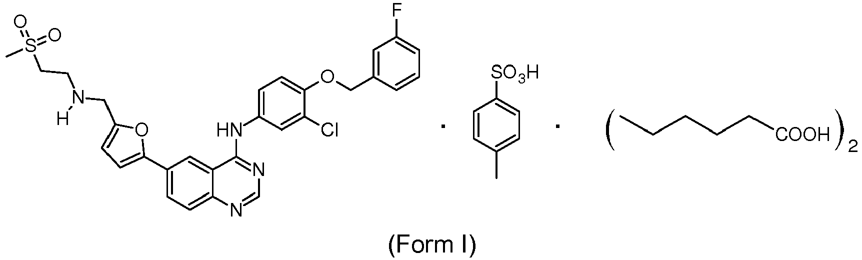
(b) Lapatinib monotosylate : caprylic acid (1:2) of formula (Form II):

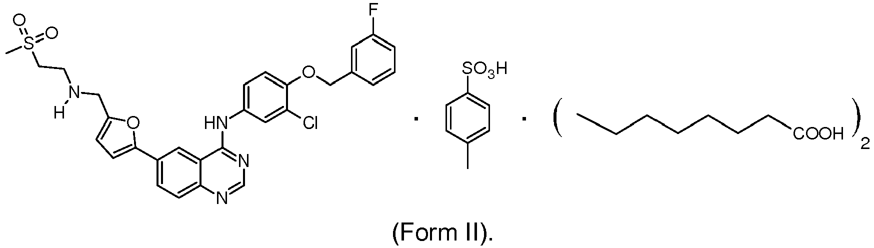

2. Co-crystal of Lapatinib according to the claim 1 being Lapatinib monotosylate : caproic acid (1:2) of formula (Form I) that has a X-Ray Powder Diffractogram (XRPD) with characteristic peaks expressed in 2 Theta value of: 18,49, 19,47, 22,81 (+/-) 0,10.

3. Co-crystal of Lapatinib according to any one of the claims from 1 to 2 being Lapatinib monotosylate : caproic acid (1:2) of formula (Form I) that has a X-Ray Powder Diffractogram (XRPD) with characteristic peaks expressed in 2 Theta value of: 4,63 (m), 7,54 (m), 15,93 (m), 18,49 (s), 19,47 (vs), 20,54 (m), 21,41 (m), 21,76 (m), 22,18 (m), 22,81 (s), 24,19 (m), 24,85 (m), 25,54 (m), 25,85 (m), 26,48 (m), (+/-) 0,10; wherein (vs) = very strong intensity, (s) = strong intensity, (m) = medium intensity.

4. Co-crystal of Lapatinib according to the claim 1 being Lapatinib monotosylate : caproic acid (1:2) of formula (Form I) having an endothermic sharp peak with an onset at 116.17°C.

5. Co-crystal of Lapatinib according to the claim 1 being Lapatinib monotosylate : caprylic acid (1:2) of formula (Form II) that has a X-Ray Powder Diffractogram (XRPD) with characteristic peaks expressed in 2 Theta value of: 18,55, 19,48, 22,71, 24,74 (+/-) 0,10.

6. Co-crystal of Lapatinib according to any one of the claims 1 and 5 being Lapatinib monotosylate : caprylic acid (1:2) of formula (Form II) that has a X-Ray Powder Diffractogram (XRPD) with characteristic peaks expressed in 2 Theta value of: 7,25 (m), 9,74 (m), 14,49 (m), 15,78 (m), 16,91 (m), 17,76 (m), 18,55 (s), 19,48 (vs), 20,16 (m), 20,93 (m), 21,58 (m), 21,88 (m), 22,71 (s), 24,06 (m), 24,74 (s), 25,34 (m), 26,08 (m), 27,52 (m), (+/-) 0,10; wherein (vs) = very strong intensity, (s) = strong intensity, (m) = medium intensity.

7. Co-crystal of Lapatinib according to the claim 1 being Lapatinib monotosylate : caprylic acid (1:2) of formula (Form II) having an endothermic sharp peak with an onset at 111.12°C.

8. Process for the preparation of the co-crystal of Lapatinib being
(a) Lapatinib monotosylate : caproic acid (1:2) of formula (Form I): 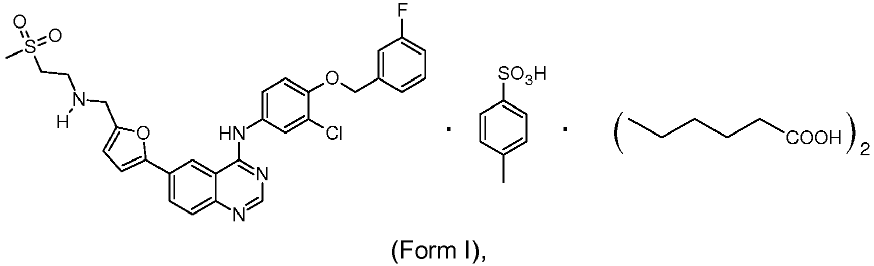 or being
(b) Lapatinib monotosylate : caprylic acid (1:2) of formula (Form II): 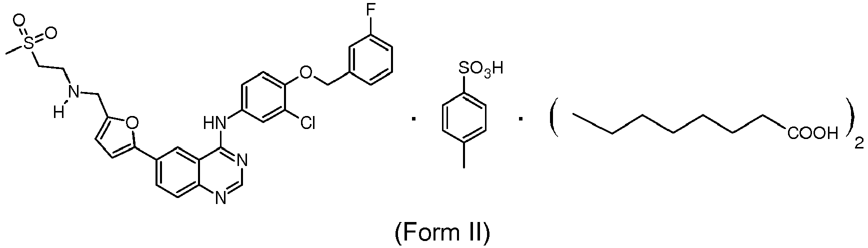 comprising the following steps:
(A.) reaction of Lapatinib of formula (I) with paratoluensulfonic acid to provide Lapatinib monotosylate of formula (I - TsOH): 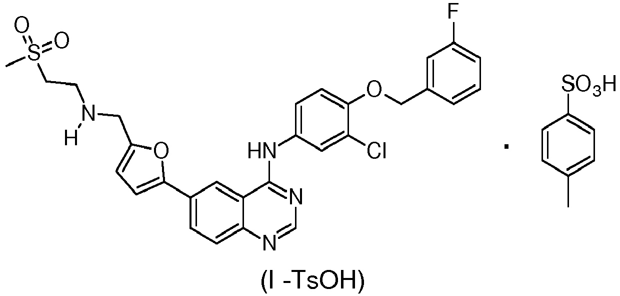
(B.) treatment of Lapatinib monotosylate respectively with caproic acid or caprylic acid.

9. Process according to the claim 8, wherein in the preparation of the co-crystal of Lapatinib monotosylate : caprylic acid (1:2) of formula (II) the solvent mixture is composed by water: EtOH 5:1.

10. Process according to the claim 8, wherein in the process for the preparation of the co-crystal of Lapatinib monotosylate : caproic acid (1:2) of formula (Form I), the amount of caproic acid is between 2.5 and 3.0 equivalents.

11. Use of Lapatinib monotosylate for the preparation of co-crystals of Lapatinib according to any one of the claims from 1 to 7.

12. Use according to the claim 11, wherein the preparation of co-crystals of Lapatinib is carried out according to the process of any one of the claim from 8 to 10.

13. Co-crystal of Lapatinib according anyone of the claims from 1 to 7 for use in medicine.

14. Co-crystal of Lapatinib according anyone of the claims from 1 to 7 for use in a method for treatment of breast cancer and other solid tumours.

15. Pharmaceutical compositions comprising the co-crystal of Lapatinib according to anyone of the claims from 1 to 7 and one or more excipients, carriers o diluents pharmaceutically acceptable.

## Patentansprüche

1. Co-Kristall von Lapatinib ausgewählt aus der Gruppe bestehend aus:
(a) Lapatinib-monotosylat : Capronsäure (1:2) der Formel (Form I): 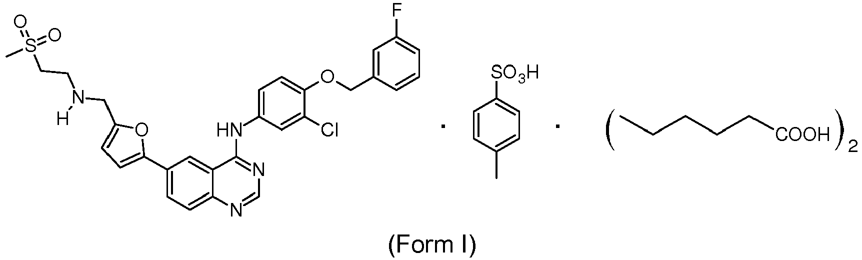
(b) Lapatinib-monotosylat : Caprylsäure (1:2) der Formel (Form II):

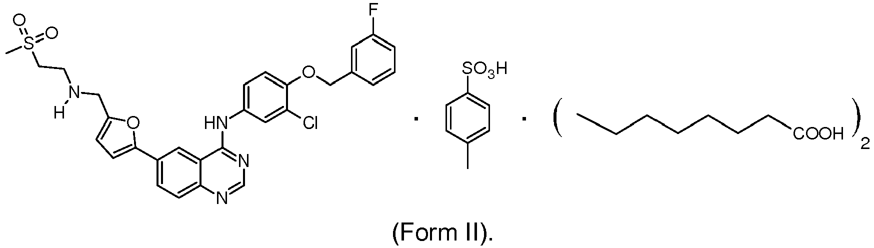

2. Co-Kristall von Lapatinib nach Anspruch 1 der Lapatinib-monotosylat : Capronsäure (1:2) der Formel (Form I) ist, der ein Pulverröntgendiffraktogramm (XRPD) mit charakteristischen Peaks aufweist, die den folgenden Werten ausgedrückt in 2 Theta entsprechen: 18,49, 19,47, 22,81 (+/-) 0,10.

3. Co-Kristall von Lapatinib nach einem der Ansprüche 1 bis 2 der Lapatinib-monotosylat : Capronsäure (1:2) der Formel (Form I) ist, der ein Pulverröntgendiffraktogramm (XRPD) mit charakteristischen Peaks aufweist, die den folgenden Werten ausgedrückt in 2 Theta entsprechen: 4,63 (m), 7,54 (m), 15,93 (m), 18,49 (s), 19,47 (vs), 20,54 (m), 21,41 (m), 21,76 (m), 22,18 (m), 22,81 (s), 24,19 (m), 24,85 (m), 25,54 (m), 25,85 (m), 26,48 (m), (+/-) 0,10; wobei (vs) = sehr starke Intensität, (s) = starke Intensität, (m) = mittlere Intensität.

4. Co-Kristall von Lapatinib nach Anspruch 1 der Lapatinib-monotosylat : Capronsäure (1:2) der Formel (Form I) mit einem endothermen scharfen Peak mit einem Beginn bei 116,17°C ist.

5. Co-Kristall von Lapatinib nach Anspruch 1 der Lapatinib-monotosylat : Caprylsäure (1:2) der Formel (Form II) ist, der ein Pulverröntgendiffraktogramm (XRPD) mit charakteristischen Peaks aufweist, die den folgenden Werten ausgedrückt in 2 Theta entsprechen: 18,55, 19,48, 22,71, 24,74 (+/-) 0,10.

6. Co-Kristall von Lapatinib nach einem der Ansprüche 1 und 5 der Lapatinibmonotosylat : Caprylsäure (1:2) der Formel (Form II) ist, der ein Pulverröntgendiffraktogramm (XRPD) mit charakteristischen Peaks aufweist, die den folgenden Werten ausgedrückt in 2 Theta entsprechen: 7,25 (m), 9,74 (m), 14,49 (m), 15,78 (m), 16,91 (m), 17,76 (m), 18,55 (s), 19,48 (vs), 20,16 (m), 20,93 (m), 21,58 (m), 21,88 (m), 22,71 (s), 24,06 (m), 24,74 (s), 25,34 (m), 26,08 (m), 27,52 (m), (+/-) 0,10; wobei (vs) = sehr starke Intensität, (s) = starke Intensität, (m) = mittlere Intensität.

7. Co-Kristall von Lapatinib nach Anspruch 1 der Lapatinib-monotosylat : Caprylsäure (1:2) der Formel (Form II) mit einem endothermen scharfen Peak mit einem Beginn bei 111,12°C ist.

8. Verfahren zur Herstellung des Co-Kristalls von Lapatinib, der
(a) Lapatinib-monotosylat : Capronsäure (1:2) der Formel (Form I): 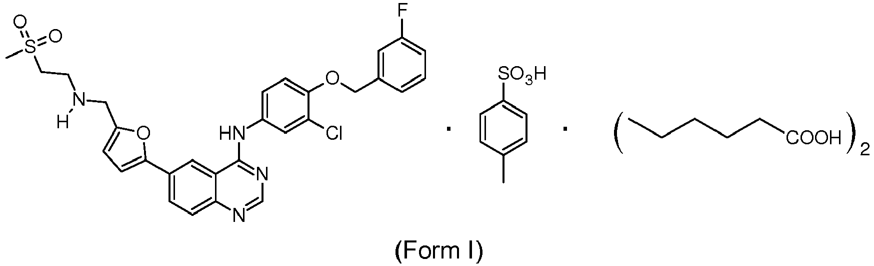
oder
(a) Lapatinib-monotosylat : Caprylsäure (1:2) der Formel (Form II): 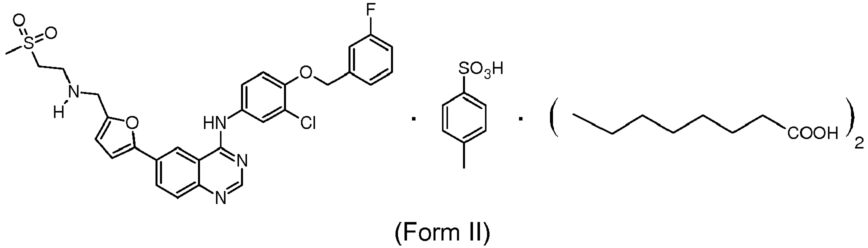
ist, umfassend die folgenden Schritte:
(A.) Reaktion von Lapatinib der Formel (I) mit para-Toluolsulfonsäure, um Lapatinib-monotosylat der Formel (I-TsOH) bereitzustellen: 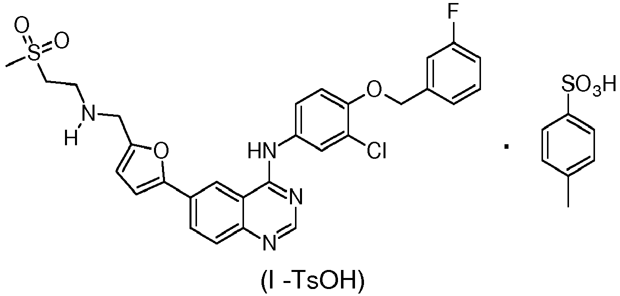
(B.) Behandlung von Lapatinib-monotosylat mit Capronsäure beziehungsweise Caprylsäure.

9. Verfahren nach Anspruch 8, wobei in der Herstellung des Co-Kristalls von Lapatinib-monotosylat : Caprylsäure (1:2) der Formel (II) die Lösungsmittelmischung aus Wasser:EtOH 5:1 zusammengesetzt ist.

10. Verfahren nach Anspruch 8, wobei in dem Verfahren zur Herstellung des Co-Kristalls von Lapatinib-monotosylat : Capronsäure (1:2) der Formel (I) die Menge an Capronsäure zwischen 2.5 und 3.0 Äquivalenten liegt.

11. Verwendung von Lapatinib-monotosylat zur Herstellung von Co-Kristallen von Lapatinib nach einem der Ansprüche 1 bis 7.

12. Verwendung nach Anspruch 11, wobei die Herstellung der Co-Kristalle von Lapatinib nach dem Verfahren gemäß einem der Ansprüche 8 bis 10 durchgeführt wird.

13. Co-Kristall von Lapatinib nach einem der Ansprüche 1 bis 7 zur Verwendung in der Medizin.

14. Co-Kristall von Lapatinib nach einem der Ansprüche 1 bis 7 zur Verwendung in einem Verfahren zur Behandlung von Brustkrebs und anderen soliden Tumoren.

15. Pharmazeutische Zusammensetzungen, die den Co-Kristall von Lapatinib nach einem der Ansprüche 1 bis 7 und einen oder mehrere pharmazeutisch annehmbare Hilfsstoffe, Träger oder Verdünnungsmittel umfassen.

## Revendications

1. Co-cristal de lapatinib choisi dans le groupe constitué de:
(a) monotosylate de lapatinib : acide caproïque (1:2) de formule (Forme I): 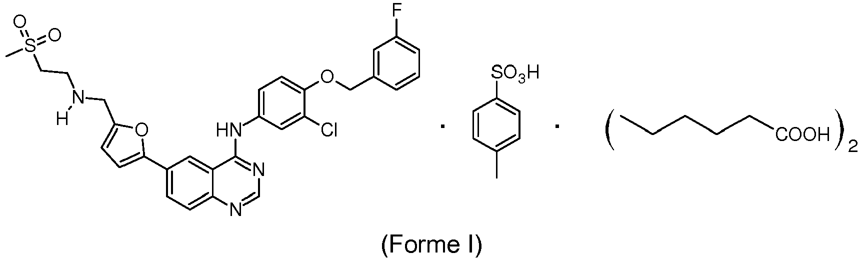
(b) monotosylate de lapatinib : acide caprylique (1:2) de formule (Forme II):

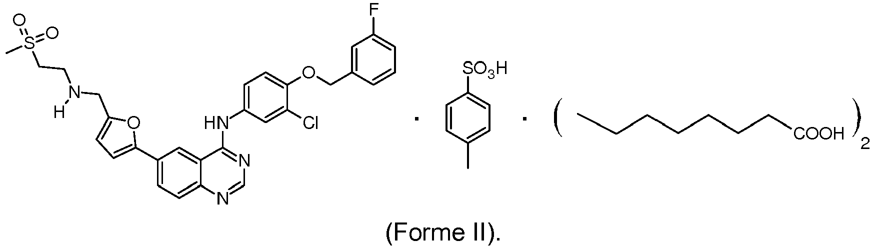

2. Co-cristal de lapatinib selon la revendication 1 qui est du monotosylate de lapatinib : acide caproïque (1:2) de formule (Forme I) qui a un diffractogramme de poudre aux rayons X (XRPD) avec des pics caractéristiques exprimés en valeur 2-thêta de : 18,49, 19,47, 22,81 (+/-) 0,10.

3. Co-cristal de lapatinib selon l'une quelconque des revendications 1 à 2 qui est du monotosylate de lapatinib : acide caproïque (1:2) de formule (Forme I) qui a un diffractogramme de poudre aux rayons X (XRPD) avec des pics caractéristiques exprimés en valeur 2-thêta de : 4,63 (m), 7,54 (m), 15,93 (m), 18,49 (s), 19,47 (vs), 20,54 (m), 21,41 (m), 21,76 (m), 22,18 (m), 22,81 (s), 24,19 (m), 24,85 (m), 25,54 (m), 25,85 (m), 26,48 (m), (+/-) 0,10; dans lequel (vs) = très forte intensité, (s) = forte intensité, (m) = intensité moyenne.

4. Co-cristal de lapatinib selon la revendication 1 qui est du monotosylate de lapatinib : acide caproïque (1:2) de formule (Forme I) possédant un pic endothermique marqué avec un début à 116,17°C.

5. Co-cristal de lapatinib selon la revendication 1 qui est du monotosylate de lapatinib : acide caprylique (1:2) de formule (Forme II) qui a un diffractogramme de poudre aux rayons X (XRPD) avec des pics caractéristiques exprimés en valeur 2-thêta de: 18,55, 19,48, 22,71, 24,74 (+/-) 0,10.

6. Co-cristal de lapatinib selon l'une quelconque des revendications 1 et 5 qui est du monotosylate de lapatinib : acide caprylique (1:2) de formule (Forme II) qui a un diffractogramme de poudre aux rayons X (XRPD) avec des pics caractéristiques exprimés en valeur 2-thêta de: 7,25 (m), 9,74 (m), 14,49 (m), 15,78 (m), 16,91 (m), 17,76 (m), 18,55 (s), 19,48 (vs), 20,16 (m), 20,93 (m), 21,58 (m), 21,88 (m), 22,71 (s), 24,06 (m), 24,74 (s), 25,34 (m), 26,08 (m), 27,52 (m), (+/-) 0,10; dans lequel (vs) = très forte intensité, (s) = forte intensité, (m) = intensité moyenne.

7. Co-cristal de lapatinib selon la revendication 1 qui est du monotosylate de lapatinib : acide caprylique (1:2) de formule (Forme II) possédant un pic endothermique marqué avec un début à 111,12°C.

8. Procédé de préparation du co-cristal de lapatinib qui est du
(a) monotosylate de lapatinib : acide caproïque (1:2) de formule (Forme I): 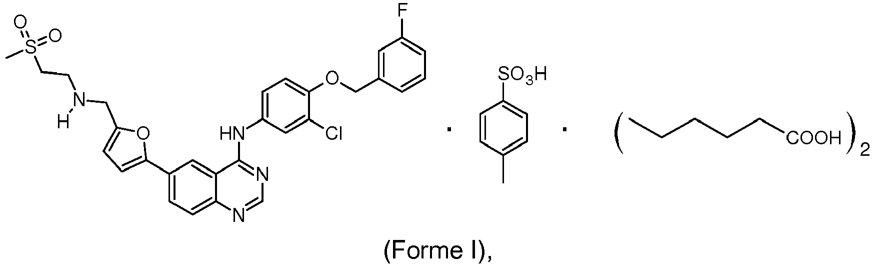 ou qui est du
(b) monotosylate de lapatinib : acide caprylique (1:2) de formule (Forme II): 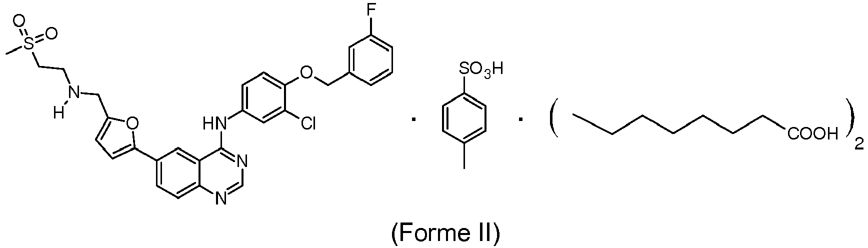
comprenant les étapes suivantes:
(A.) réaction de lapatinib de formule (I) avec de l'acide paratoluène-sulfonique pour fournir du monotosylate de lapatinib de formule (I - TsOH): 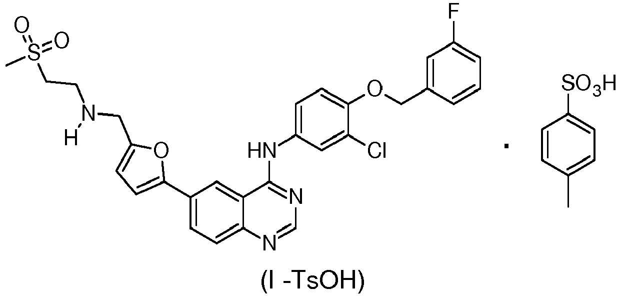
(B.) traitement du monotosylate de lapatinib respectivement avec de l'acide caproïque ou de l'acide caprylique.

9. Procédé selon la revendication 8, dans lequel, dans la préparation du co-cristal de monotosylate de lapatinib : acide caprylique (1:2) de formule (II) le mélange de solvants est composé d'eau : EtOH 5:1.

10. Procédé selon la revendication 8, dans lequel, dans le procédé de préparation du co-cristal de monotosylate de lapatinib : acide caproïque (1:2) de formule (Forme I), la quantité d'acide caproïque est comprise entre 2.5 et 3.0 équivalents.

11. Utilisation de monotosylate de lapatinib pour la préparation de co-cristaux de lapatinib selon l'une quelconque des revendications 1 à 7.

12. Utilisation selon la revendication 11, dans laquelle la préparation de co-cristaux de lapatinib est effectuée selon le procédé de l'une quelconque des revendications 8 à 10.

13. Co-cristal de lapatinib selon l'une quelconque des revendications 1 à 7 pour une utilisation en médecine.

14. Co-cristal de lapatinib selon l'une quelconques des revendications 1 à 7 pour une utilisation dans une méthode de traitement du cancer du sein et d'autres tumeurs solides.

15. Compositions pharmaceutiques comprenant le co-cristal de lapatinib selon l'une quelconque des revendications 1 à 7 et un ou plusieurs excipients, véhicules ou diluants acceptables sur le plan pharmaceutique.
